# EUROPÄISCHE PATENTANMELDUNG

(11) **EP 1 394 265 A2**
(43) Veröffentlichungstag der Anmeldung: **03.03.2004**
(21) Anmeldenummer: 03016464.4
(22) Anmeldetag: 22.07.2003
(51) Int. Cl.: C12Q 1/533, C12Q 1/02, C12N 9/90, C12Q 1/68

(54) **Verfahren zur Identifikation von Fungiziden unter Verwendung einer Ribose-5-Phosphat Isomerase**

(30) Priorität: 01.08.2002 DE 10235129
(71) Anmelder: Bayer CropScience AG, 40789 Monheim (DE)
(72) Erfinder: Schreier, Peter, Prof. Dr., 50674 Köln (DE); Leuthner, Birgitta, Dr., 40764 Langenfeld (DE); Li, Volhart, Dr., 42553 Velbert (DE); Kuck, Karl-Heinz, Dr., 40764 Langenfeld (DE); Dunkel, Ralf, Dr., 40789 Monheim (DE)

(57) **Zusammenfassung**

Die vorliegende Erfindung betrifft ein Verfahren zum Identifizieren von Fungiziden, die Verwendung von Ribose-5-phosphat-Isomerase zum Identifizieren von Fungiziden, die Verwendung von Inhibitoren der Ribose-5-phosphat-Isomerase als Fungizide sowie Nukleinsäuren, die für ein Polypeptid mit der Aktivität einer Ribose-5-phosphat-Isomerase aus pflanzenpathogenen Pilzen kodieren.

SEQUENCE LISTING

<110> Bayer AG

<120> Verfahren zum Identifizieren von Fungiziden

<130> Le A 36 055

<160> 2

<170> PatentIn version 3.1

<210> 1

<211> 1020

<212> DNA

<213> Ustilago maydis

<220>

<221> CDS

<222> (1)..(1017)

<223>

<400> 1
atg caa tcc agg ctt ctc tct ctg gtt tca tcc cat ctt gtg tct aag 48
Met Gln Ser Arg Leu Leu Ser Leu Val Ser Ser His Leu Val Ser Lys
1               5                   10                  15

cgc tgt ttc atc gct cgc agt gct gcc ctt gcg ccg ctt ctc ctt cac 96
Arg Cys Phe Ile Ala Arg Ser Ala Ala Leu Ala Pro Leu Leu Leu His
            20                  25                  30

ccc caa cgt cta cgt ctc act tgc cct cgc tcc ttt tct tct caa caa 144
Pro Gln Arg Leu Arg Leu Thr Cys Pro Arg Ser Phe Ser Ser Gln Gln
        35                  40                  45

tct ggc cca cgc aag atg gct tct tcc aac gct acc aac agt acc agt 192
Ser Gly Pro Arg Lys Met Ala Ser Ser Asn Ala Thr Asn Ser Thr Ser
    50                  55                  60

gcc gcc agt gct gcc aac acc aac tcg tct gct ttc aag agt gcc gaa 240

Ala Ala Ser Ala Ala Asn Thr Asn Ser Ser Ala Phe Lys Ser Ala Glu
65                  70                  75                  80

ttg gct gct ttg agc ggc gtc gag gct gct aag cgt gct gct gcg tac     288
Leu Ala Ala Leu Ser Gly Val Glu Ala Ala Lys Arg Ala Ala Ala Tyr
                85                  90                  95

gct gcc gtt gac aac cac gtc aag ccg cag cac gag atc att ggt att     336
Ala Ala Val Asp Asn His Val Lys Pro Gln His Glu Ile Ile Gly Ile
            100                 105                 110

ggc tcg ggc tca acg gtg cct tat gtt gtt gag cga att gct caa cag     384
Gly Ser Gly Ser Thr Val Pro Tyr Val Val Glu Arg Ile Ala Gln Gln
        115                 120                 125

gga cct gct gtg aat gca aag cgt tgg ttc gtt ccc acc ggc ttc cag     432
Gly Pro Ala Val Asn Ala Lys Arg Trp Phe Val Pro Thr Gly Phe Gln
    130                 135                 140

tca cgc gaa ctc atc atc aac gcc ggc ctt cgt ctc ggt gat gtg gat     480
Ser Arg Glu Leu Ile Ile Asn Ala Gly Leu Arg Leu Gly Asp Val Asp
145                 150                 155                 160

agc ttc ccc agc atc gac gtc act atc gat ggc gct gac gag gtc gac     528
Ser Phe Pro Ser Ile Asp Val Thr Ile Asp Gly Ala Asp Glu Val Asp
                165                 170                 175

aat gcg ctc aac tgc atc aag ggc ggc ggt gct tgt cat ttg cgc gaa     576
Asn Ala Leu Asn Cys Ile Lys Gly Gly Gly Ala Cys His Leu Arg Glu
            180                 185                 190

aag gta ctc gcc gaa gcc gcc aat gaa ttt gtc gta gtc gct gac tac     624
Lys Val Leu Ala Glu Ala Ala Asn Glu Phe Val Val Val Ala Asp Tyr
        195                 200                 205

cgc aag aat gga tcg cag ctc ggc aca aag tgg ctg caa ggt gtc ccc     672
Arg Lys Asn Gly Ser Gln Leu Gly Thr Lys Trp Leu Gln Gly Val Pro
    210                 215                 220

atc gag gtc gct ccg ttt gcc tat gca aaa gtg ctt cag aac ctc aaa     720
Ile Glu Val Ala Pro Phe Ala Tyr Ala Lys Val Leu Gln Asn Leu Lys
225                 230                 235                 240

aag atg ggt tct gac aag gcg gtc ctt cgc atg ggc aag gca aaa gcc     768
Lys Met Gly Ser Asp Lys Ala Val Leu Arg Met Gly Lys Ala Lys Ala
                245                 250                 255

ggt ccc gtc gtt aca gat aat ggc aac ttt tgc atc gat gct ccc ttc 816
Gly Pro Val Val Thr Asp Asn Gly Asn Phe Cys Ile Asp Ala Pro Phe
            260                 265                 270

ccc gaa gca cag atg aag gat ccc tct gat ttg ctc aag cgt atc aag 864
Pro Glu Ala Gln Met Lys Asp Pro Ser Asp Leu Leu Lys Arg Ile Lys
        275                 280                 285

ttg ctc acc ggt gta ctt gag gtc ggt ctg ttt tgc aac att tgc aag 912
Leu Leu Thr Gly Val Leu Glu Val Gly Leu Phe Cys Asn Ile Cys Lys
    290                 295                 300

tcc gcc tac ttt ggc aac gat gac ggc acc atc acc atc aaa acc gcc 960
Ser Ala Tyr Phe Gly Asn Asp Asp Gly Thr Ile Thr Ile Lys Thr Ala
305                 310                 315                 320

gcc gga gat gtg caa gag ggc gtc cac ttt gac gtc tcc aag gcg cct 1008
Ala Gly Asp Val Gln Glu Gly Val His Phe Asp Val Ser Lys Ala Pro
                325                 330                 335

gca aca gca taa 1020
Ala Thr Ala

<210> 2

<211> 339

<212> PRT

<213> Ustilago maydis

<400> 2

Met Gln Ser Arg Leu Leu Ser Leu Val Ser Ser His Leu Val Ser Lys
1               5                   10                  15

Arg Cys Phe Ile Ala Arg Ser Ala Ala Leu Ala Pro Leu Leu Leu His
            20                  25                  30

Pro Gln Arg Leu Arg Leu Thr Cys Pro Arg Ser Phe Ser Ser Gln Gln
        35                  40                  45

Ser Gly Pro Arg Lys Met Ala Ser Ser Asn Ala Thr Asn Ser Thr Ser
    50                  55                  60

Ala Ala Ser Ala Ala Asn Thr Asn Ser Ser Ala Phe Lys Ser Ala Glu
65                  70                  75                  80

Leu Ala Ala Leu Ser Gly Val Glu Ala Ala Lys Arg Ala Ala Ala Tyr
                85                  90                  95

Ala Ala Val Asp Asn His Val Lys Pro Gln His Glu Ile Ile Gly Ile
            100                 105                 110

Gly Ser Gly Ser Thr Val Pro Tyr Val Val Glu Arg Ile Ala Gln Gln
        115                 120                 125

Gly Pro Ala Val Asn Ala Lys Arg Trp Phe Val Pro Thr Gly Phe Gln
    130                 135                 140

Ser Arg Glu Leu Ile Ile Asn Ala Gly Leu Arg Leu Gly Asp Val Asp
145                 150                 155                 160

Ser Phe Pro Ser Ile Asp Val Thr Ile Asp Gly Ala Asp Glu Val Asp
                165                 170                 175

Asn Ala Leu Asn Cys Ile Lys Gly Gly Gly Ala Cys His Leu Arg Glu
            180                 185                 190

Lys Val Leu Ala Glu Ala Ala Asn Glu Phe Val Val Val Ala Asp Tyr
        195                 200                 205

Arg Lys Asn Gly Ser Gln Leu Gly Thr Lys Trp Leu Gln Gly Val Pro
    210                 215                 220

Ile Glu Val Ala Pro Phe Ala Tyr Ala Lys Val Leu Gln Asn Leu Lys
225                 230                 235                 240

Lys Met Gly Ser Asp Lys Ala Val Leu Arg Met Gly Lys Ala Lys Ala
                245                 250                 255

Gly Pro Val Val Thr Asp Asn Gly Asn Phe Cys Ile Asp Ala Pro Phe
            260                 265                 270

Pro Glu Ala Gln Met Lys Asp Pro Ser Asp Leu Leu Lys Arg Ile Lys
        275                 280                 285

Leu Leu Thr Gly Val Leu Glu Val Gly Leu Phe Cys Asn Ile Cys Lys
    290                 295                 300

Ser Ala Tyr Phe Gly Asn Asp Asp Gly Thr Ile Thr Ile Lys Thr Ala
305                 310                 315                 320

Ala Gly Asp Val Gln Glu Gly Val His Phe Asp Val Ser Lys Ala Pro
                325                 330                 335

Ala Thr Ala

## Beschreibung

Die vorliegende Erfindung betrifft ein Verfahren zum Identifizieren von Fungiziden, die Verwendung von Ribose-5-phosphat-Isomerase zum Identifizieren von Fungiziden, die Verwendung von Inhibitoren der Ribose-5-phosphat-Isomerase als Fungizide sowie Nukleinsäuren, die für ein Polypeptid mit der Aktivität einer Ribose-5-phosphat-Isomerase aus pflanzenpathogenen Pilzen kodieren.

Unerwünschtes Pilzwachstum, das in der Landwirtschaft jedes Jahr zu beträchtlichen Schäden führt, kann. durch die Verwendung von Fungiziden kontrolliert werden. Die Ansprüche an Fungizide sind dabei hinsichtlich ihrer Wirksamkeit, Kosten und vor allem ihrer Umweltverträglichkeit stetig angestiegen. Es existiert deshalb ein Bedarf an neuen Substanzen bzw. Substanzklassen, die zu leistungsfähigen und umweltverträglichen neuen Fungiziden entwickelt werden können. Im Allgemeinen ist es üblich, in Gewächshaustests nach solchen neuen Leitstrukturen zu suchen. Solche Tests sind jedoch arbeitsintensiv und teuer. Die Anzahl der Substanzen, die im Gewächshaus getestet werden können, ist entsprechend begrenzt. Eine Alternative zu solchen Tests ist die Verwendung von sogenannten Hochdurchsatzverfahren (HTS = high throughput screening). Dabei werden in einem automatisierten Verfahren eine große Anzahl von Einzelsubstanzen hinsichtlich ihrer Wirkung auf Zellen, individuelle Genprodukte oder Gene getestet. Wird für bestimmte Substanzen eine Wirkung nachgewiesen, so können diese in herkömmlichen Screeningverfahren untersucht und gegebenenfalls weiter entwickelt werden.

Vorteilhafte Angriffspunkte für Fungizide werden oft in essentiellen Biosynthesewegen gesucht. Ideale Fungizide sind weiterhin solche Stoffe, die Genprodukte hemmen, die eine entscheidende Bedeutung bei der Ausprägung der Pathogenität eines Pilzes haben.

Aufgabe der vorliegenden Erfindung war es deshalb, einen geeigneten neuen Angriffspunkt für potentielle fungizide Wirkstoffe zu ïdentifizieren, zugänglich zu machen, und ein Verfahren zur Verfügung zu stellen, das die Identifizierung von Modulatoren dieses Angriffspunkts ermöglicht, die als Fungizide verwendet werden können.

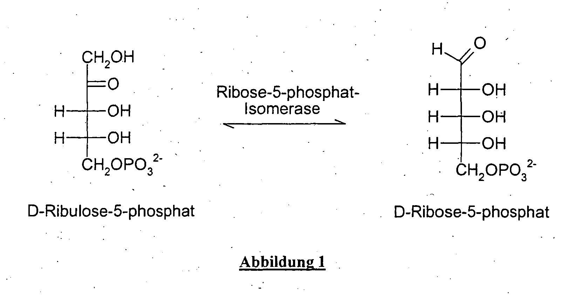

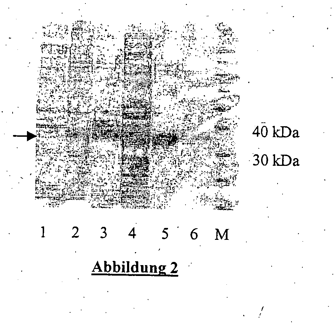

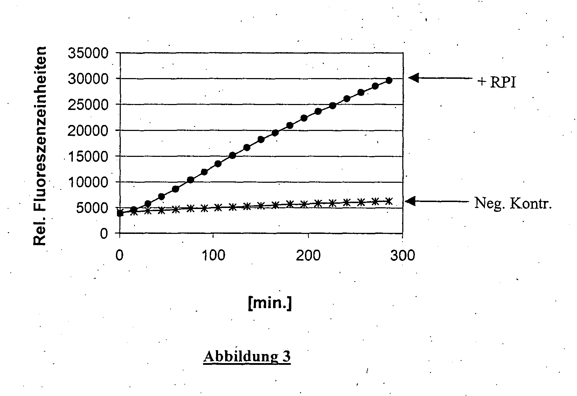

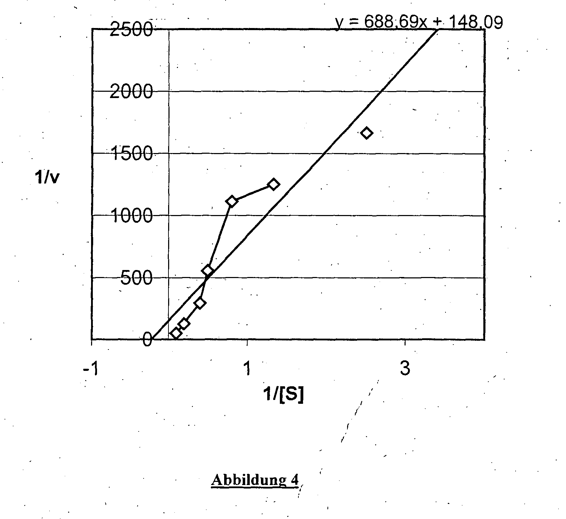

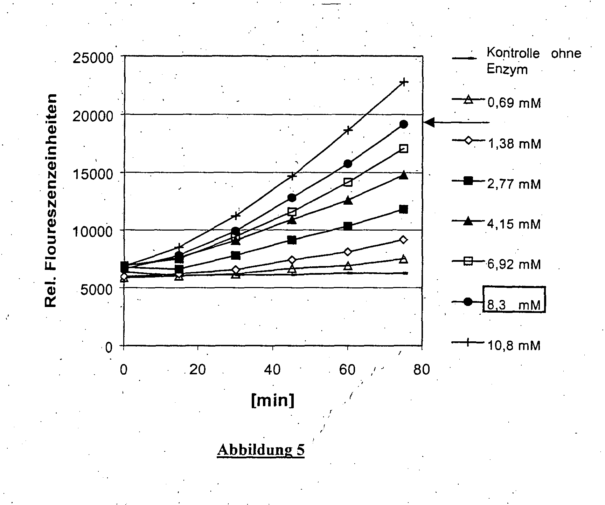

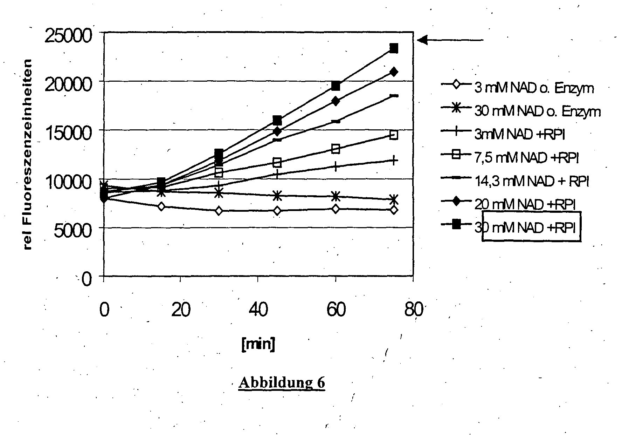

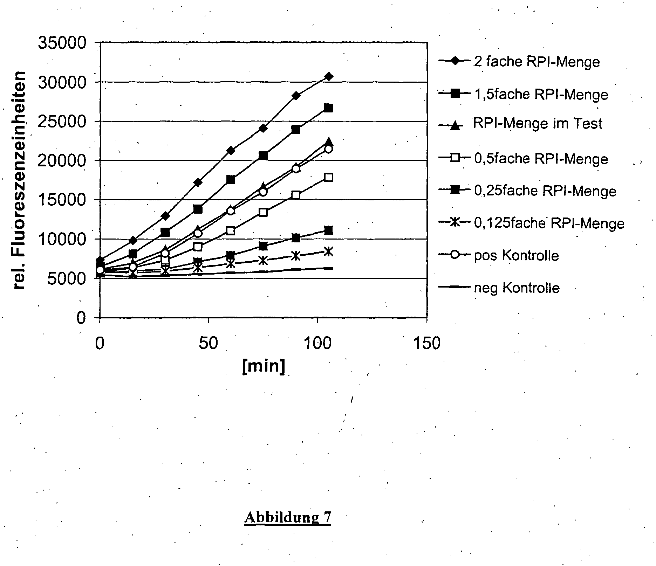

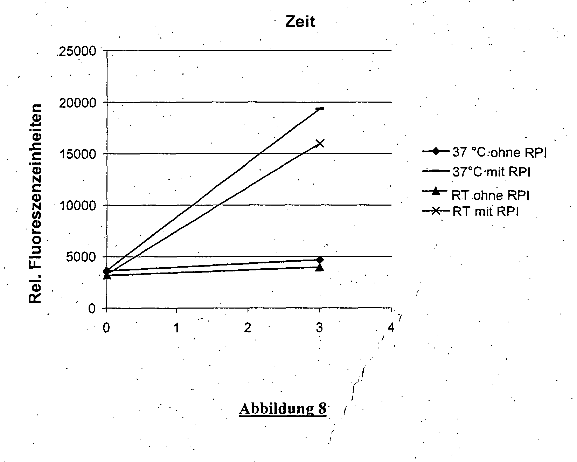

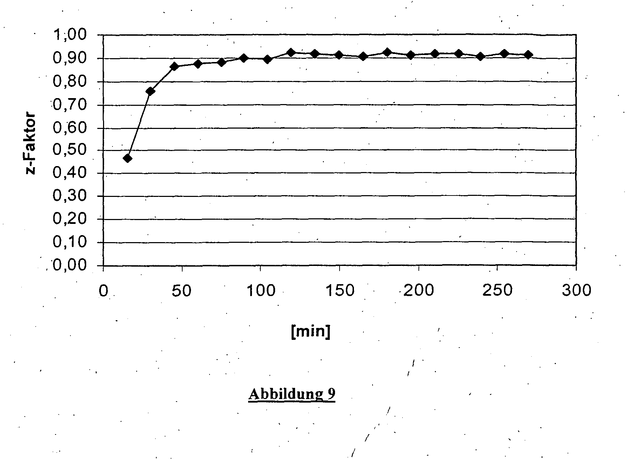

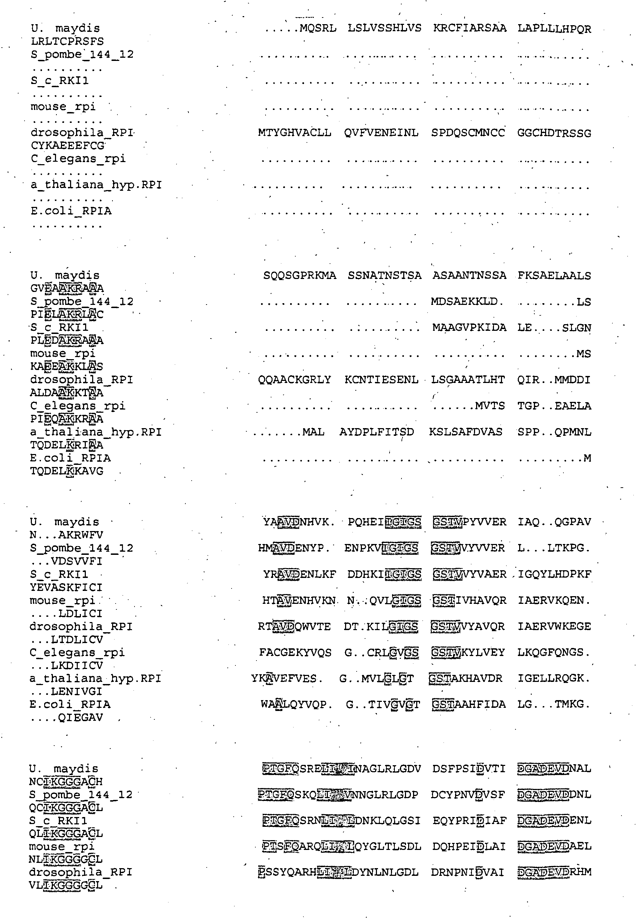

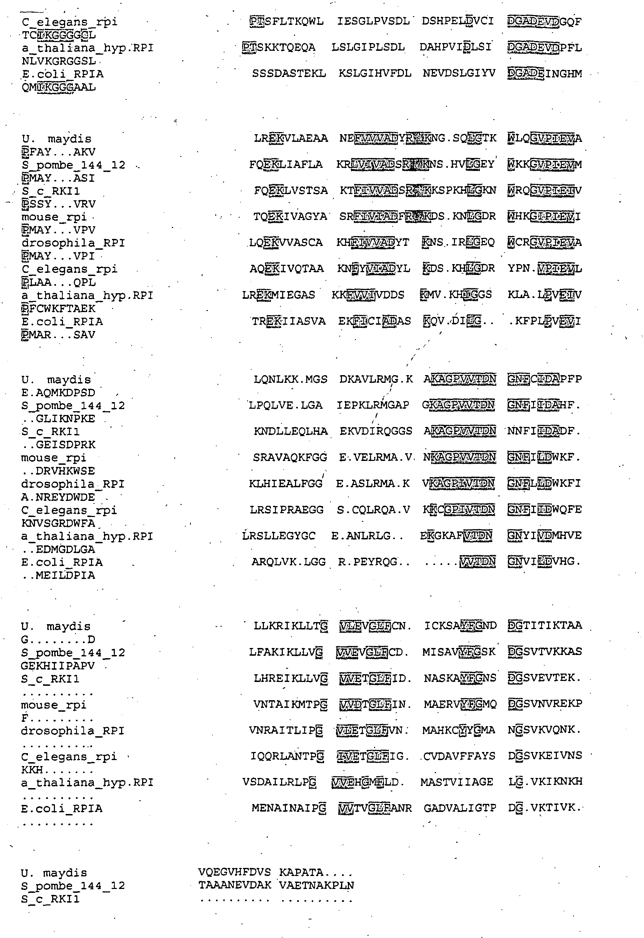

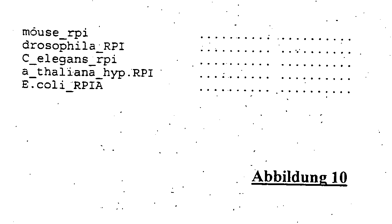

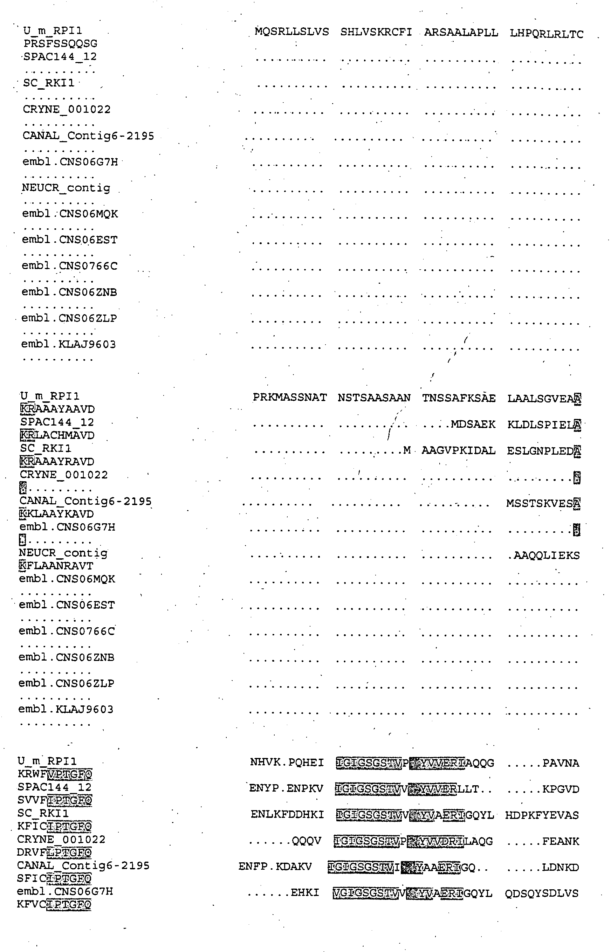

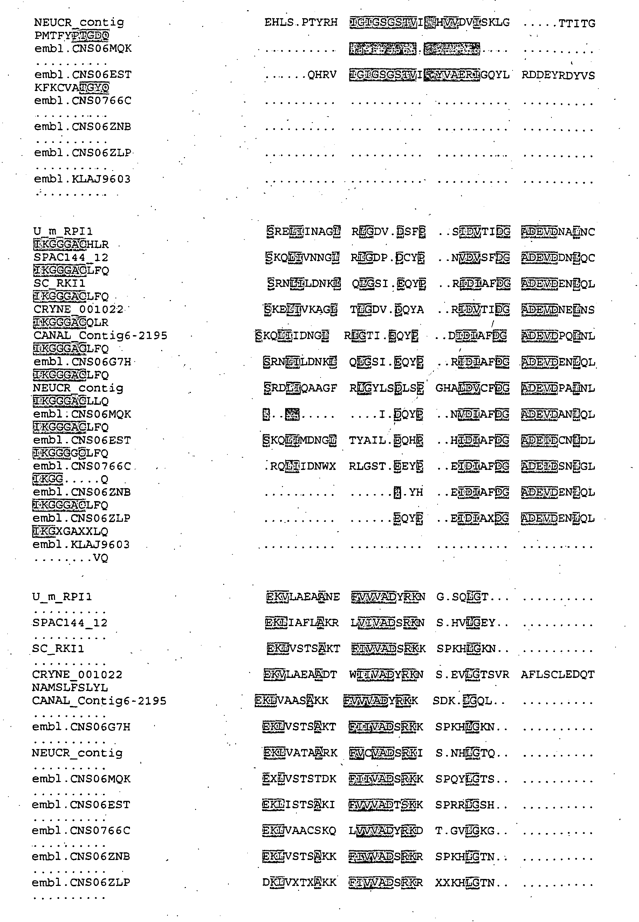

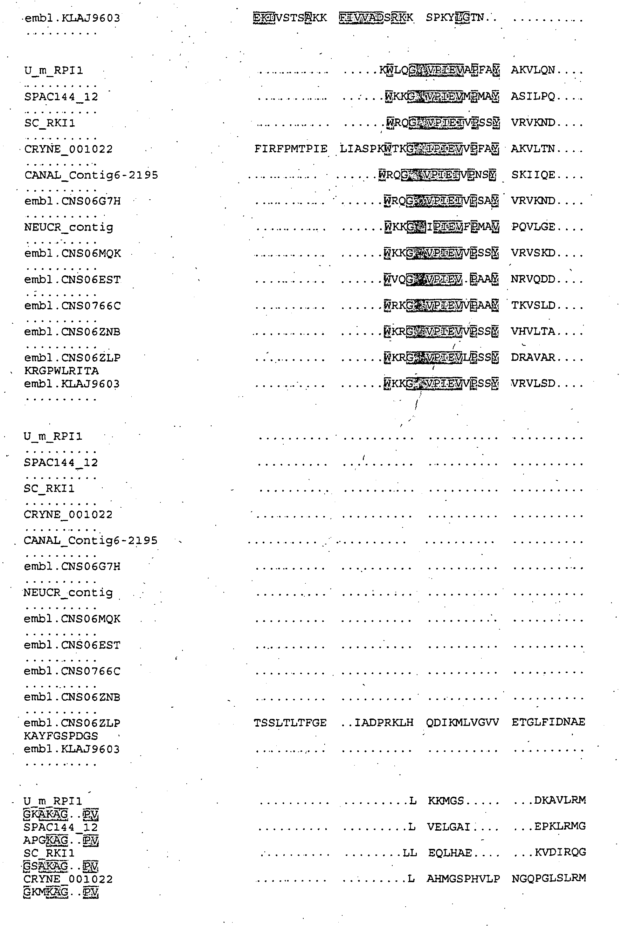

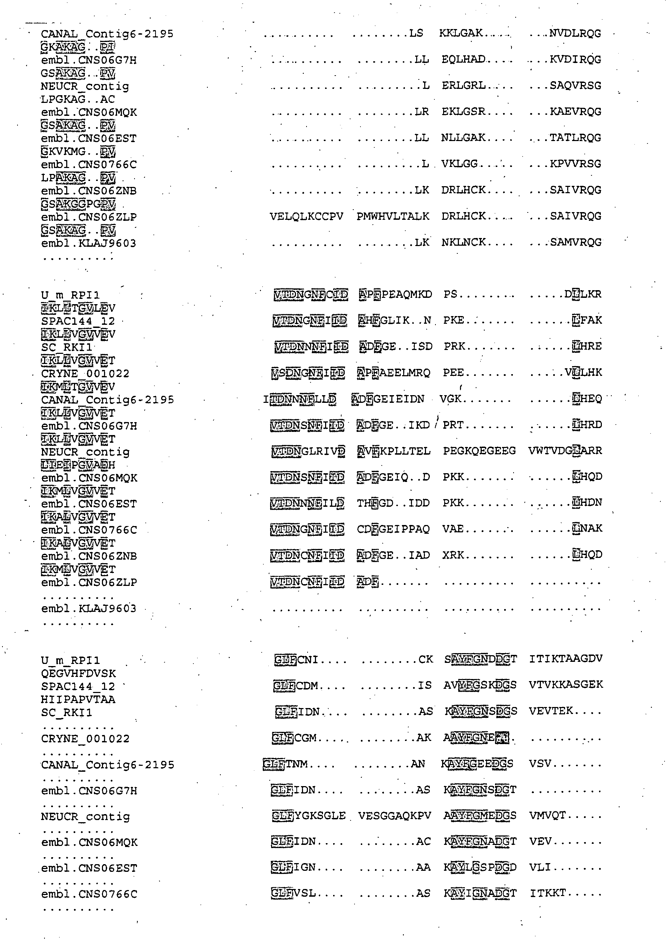

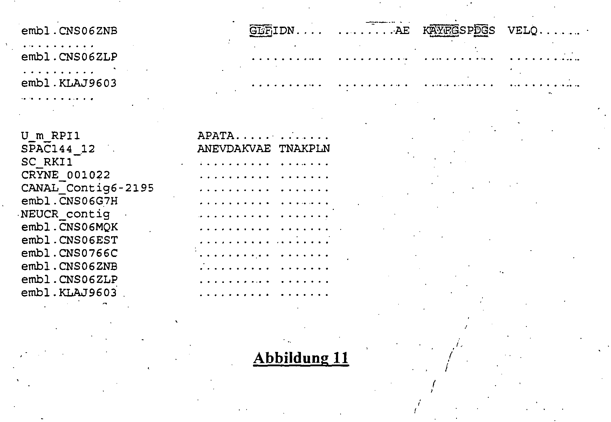

Es wurde nun gefunden, dass die Ribose-5-phosphat-Isomerase aus Pilzen verwendet werden kann, um in geeigneten Verfahren Wirkstoffe zu identifizieren, die als Fungizide eingesetzt werden können. Die cytoplasmatische Ribose-5-phosphat-Isomerase, im Folgenden auch mit "RPI" abgekürzt, die auch als Phosphopentoseisomerase, Phosphoriboisomerase, Ribose-phosphat-Isomerase, 5-Phosphoribose-Isomerase oder D-Ribose-5-phosphat-Isomeräse bezeichnet wird, ist das erste Enzym im nicht-oxidativen Zweig des Pentose-phosphat-Wegs (EC 5.3.1.6). In diesem Abschnitt des Stoffwechsels wird das Verhältnis zwischen den Pentosen und den Hexosen im Organismus eingestellt und wichtige Zucker-Grundbausteine ineinander überführt. Durch die RPI wird aus Ribulose-5-phosphat, dem Produkt des oxidativen Zweigs des Pentosephosphatwegs, Ribose-5-phosphat gewonnen, das als Grundbaustein für die Pyrimidin-, Purin-, Tryptophan- und Histidin-Biosynthese benötigt wird (Abbildung 1).

Ribose-5-phosphat wird im nächsten Schritt zu Phosphoribosylpyrophosphat aktiviert und dient dann als Ausgangsverbindung für die zuvor angeführten Stoffwechselprodukte und als Grundbaustein für den so genannten Salvage-Pathway der Nukleinsäuren. Auf gleiche Weise katalysiert die RPI die Reaktion in die Gegenrichtung, sodass aus Ribose-5-phosphat Ribulose-5-phosphat erhalten wird, das z.B. in der Pflanze als Substrat für die Phosphoribulose-Kinase (Calvin-Zyklus) dient. Im Pentosephosphat-Weg entstehen in drei weiteren Schritten aus Ribose-5-phosphat durch Transketolase, Transaldolase und Ribulose-5-phosphat-Epimerase die Zuckerbausteine Sedoheptulose-7-phosphat, Glycerinaldehyd-3-phosphat, Erythrose-4-phosphat und Fructose-6-phosphat.

Das Enzym Ribose-5-phosphat-Isomerase ist bereits seit langer Zeit bekannt. Dafür kodierende Nukleinsäuren wurden bereits aus zahlreichen Bakterien sowie aus Insekten, wie z.B. C. elegans, und Pflanzen, wie z.B. A. *thaliana,* isoliert. Aus Pilzen sind nur sehr wenige für Ribose-5-phosphat-Isomerasen kodierende Nukleinsäuren bekannt geworden. In S. *cerevisiae* ist nur ein für das Polypeptid kodierendes RPI-Gen, *rkil,* vorhanden, dessen Knock-out, also Inaktivierung, als letal beschrieben wird (T. Miosga und F.K. Zimmermann, *Curr. Genet.* 30 (1996) 404-409; R. Reuter, M. Naumann, J. Bar, T. Migosa und G. Kopperschlager, *Bioseparation* 7(2) (1998) 107-115 ). Neben S. *cerevisiae* ist die RPI nur noch aus wenigen weiteren Hefen bekannt, so z.B. aus S. *pombe* oder *S. kluyveri.* Aus pflanzenpathogenen Pilzen wurden dagegen bislang keine für eine Ribose-5-phosphat-Isomerase kodierenden Nukleinsäuren isoliert. Weder in den genannten. Hefen noch in human- oder pflanzenpathogenen Pilzen wurde jedoch bislang untersucht, ob die Ribose-5-phosphat-Isomerase gezielt durch eine chemische Verbindung inhibiert werden kann, und ob eine solche Inhibition auch *in vivo,* d.h. bei Pilzen als solchen, erzielt werden kann. Es wurde ebenso bislang noch nicht erforscht, ob die Inhibition der Ribose-5-phosphat-Isomerase durch einen Wirkstoff den Pilz so weit schädigt oder tötet, dass man den Wirkstoff als Fungizid verwenden kann, sich die Ribose-5-phosphat-Isomerase also als Zielprotein für fungizide Wirkstoffe eignet. Die Tatsache, dass der Knock out des entsprechenden Gens in *S. cerevisae* als letal eingestuft wurde, kann nur als Indiz dafür betrachtet werden, dass sich das davon kodierte Genprodukt als Zielprotein für Fungizide eignet.

Aufgabe der vorliegenden Erfindung war es deshalb auch, eine RPI aus einem pflanzenpathogenen Pilz zur Verfügung zu stellen, um mit Hilfe dieses Polypeptids die vorstehend formulierte Aufgabe zu lösen.

### Abbildungen und Sequenzprotokoll

### Abbildung 1

Die enzymatische Aktivität der Ribose-5-phosphat-Isomerase (RPI). D-Ribulose-5-phosphat wird in D-Ribose-5-phosphat umgewandelt.

### Abbildung 2

Heterologe Expression und Reinigung von RPI1 aus *U. maydis.* Spur 1: nichtinduzierte Zellen; Spur 2: Zellen nach der Induktion; Spur 3: Niederschlag nach Zellaufschluss; Spur 4: Überstand nach Zellaufschluss; Spur 5: Elutionsfraktion von der Ni-Säule; Spur 6: Fraktion nach der PD-Säule; M: 10 kDa-Proteinstandard.

### Abbildung 3

Kinetik der NADH-Zunahme im dreifach gekoppelten Testsystem. Das Assayvolumen betrug 35 µl. Ribose-5-phosphat wurde in einer Konzentration von 8,3 mM eingesetzt. Es wurden 25 ng RPI verwendet. Zusätzlich wurde eine Negativ-Kontrolle (Neg. Kontr.) durchgeführt, wobei keine RPI enthalten war. Die Entstehung von NADH kann anhand der zunehmenden relativen Fluoreszenz verfolgt werden.

### Abbildung 4

Lineweaver-Burk-Plot zur K_{M}-Wert-Bestimmung. Für das Substrat der RPI, Ribose-5-phosphat, wurde der K_{M}-Wert mittels der Absorptionszunahme bei 290 nm bestimmt, d.h. ohne Zuhilfenahme der gekoppelten Enzyme. Die Messung wurde mit 400 ng RPI durchgeführt. Im Diagramm ist 1/v in [min/mM] gegen 1/[S] in [1/mM] aufgetragen.

### Abbildung 5

Messung der Entstehung von NADH in Abhängigkeit von der eingesetzten Ribose-5-phosphat-Konzentration (im Diagramm angegeben) bei einer eingesetzten RPI-Menge von 25 ng in einem Reaktionsvolumen von 35 µl. Eine Konzentration von 8,3 mM erwies sich als besonders geeignet (vgl. Pfeil und Rahmen).

### Abbildung 6

Bestimmung der Fluoreszenzzunahme in Abhängigkeit von der Konzentration an NAD im erfindungsgemäßen Verfahren zur Identifizierung von Fungiziden. Das Assayvolumen betrug 35 µl. Es wurden 8,3 mM Ribose-5-phosphat eingesetzt.

### Abbildung 7

Durchführung des erfindungsgemäßen Verfahrens zur Identifizierung von Fungiziden mit unterschiedlichen Mengen an RPI ausgehend von 25 ng RPI/well (Positiv-Kontrolle). Die Negativ-Kontrolle entspricht dem Ansatz ohne Zugabe von RPI.

### Abbildung 8

Temperaturabhängigkeit der RPI-Reaktion. Die Reaktion und die Kontrolle ohne RPI wurde für jeweils 3 Stunden bei Raumtemperatur (RT) bzw. bei 37°C inkubiert.

### Abbildung 9

Bestimmung des z-Faktors des erfindungsgemäßen Verfahrens basierend auf Fluoreszenzmessungen bei 24 Versuchsansätzen. Der z-Faktor ist eine Größe zur Bestimmung der Qualität eines Screeningverfahrens bzw. eines Hemmtests. In den z-Faktor gehen neben der Differenz aus Signal und Hintergrund auch die Streuung aller Messwerte in die Berechnung mit ein.

### Abbildung 10

Alignment der RPI aus *U. maydis* mit bekannten Ribose-Phosphat-Isomerase-Sequenzen aus *S. pombe, S. cerevisiae, Mus musculus, D. melanogaster, C. elegans, A. thaliana* und *E. coli.* Identische Aminosäuren sind grau unterlegt.

### Abbildung 11

Alignment der RPI aus *U. maydis* mit Ribose-Phosphat-Isomerase-Sequenzen aus anderen Pilzen (U.m. = *U. maydis;* SPAC144_12 = S. *pombe*; SC_RKI1 = *S*. *cerevisiae;* CRYNE_001022 = *C. neoformans;* CANAL_Contig6-2195 = *C. albicans;* embl.CNS06G7H = *S. bayanus;* NEUCR_contig = *N. crassa;* embl.CNS06MQK = *S. kluyveri;* embl.CNS06EST = *Z. rouxii*; embl.CNS0766C = *P. angusta;* embl.CNS06ZNB = *K. marxianus*, embl.CNS06ZLP = *K. marxianus*; embl.KLAJ9603 *= K. lactis).* Einige der gezeigten Sequenzen waren bislang noch nicht annotiert, d.h. es war bislang nicht bekannt, dass diese Sequenzen für eine RPI kodieren. Es handelt sich dabei um die Sequenzen aus *Cryptococcus neoformans, Candida albicans, Neurospora crassa, Zygosaccharomyces rouxii* und auch die für eine RPI kodierende Sequenz aus *Hypocrea jecorina* (BM076780). Für die Sequenzen aus *S. pombe, S. bayanus, S. cerevisiae, S. kluyveri,. P. angusta, Kluyveromyces marxianus, K. lactis* und z.B. auch *Yarrowia lipolytica* (embl|CNS06QCU), allesamt nicht pflanzenpathogene Pilze, war bereits bekannt, dass diese für eine RPI kodieren. Identische Aminosäuren sind grau unterlegt

### Abbildung 12

Ausschnitt aus einer 384-er Mikrotiterplatte, in der die Umsetzung von D-Ribose-5-phosphat zu D-Ribulose-5-phoshat durch den Nachweis der Ribulose als Purpurfarbstoff (546 nm) nach einer Farbreaktion mit einer Carbazol/Cystein/HCl-Lösung verfolgt wurde. In den Zeilen sind von oben nach unten abnehmende Mengen an Ribose-5-phosphat eingesetzt worden; Spalte 1 zeigt die Reaktion in Abwesenheit von RPI1, Spalte 2 und 3 in Gegenwart von 400 ng RPI1.

### Abbildung 13

Nachweis der enzymatischen Reaktion von RPI mittels eines gekoppelten Enzymtests. Der Nachweis erfolgt hier fluorometrisch über die NADH-Abnahme. Bei Anwesenheit des Enzyms wird NADH verbraucht, was in einer Abnahme der relativen Fluoreszenz resultiert (vgl. Bsp. 2C).

### Abbildung 14

Sporenanalyse der rpi-Knock-out-Stämme. Die mit **A** bezeichneten Platten zeigen Sporen auf Vollmedium-Platten ohne Selektion. Die mit **B** bezeichneten Platten enthalten Hygromycin, weshalb hier nur Sporen wachsen können, die eine Resistenz aufgrund der Anwesenheit der Resistenz Kassette besitzen. Die auf den Selektionsplatten **B** wachsenden Sporen wurden darauf untersucht, ob sie diploid sind, und durch PCR das etwaige Vorhandensein des Wildtyp-Gens *rpi* kontrolliert.

### SEQ ID NO. 1:

Die für die RPI kodierende cDNA-Sequenz aus *Ustilago maydis.*

### SEQ ID NO. 2:

Die Aminosäuresequenz der RPI aus *Ustilago maydis,* kodiert von der Sequenz gemäß SEQ ID NO. 1.

### Definitionen

Der Begriff "Identität", wie er hierin verwendet wird, bezieht sich auf die Zahl von Sequenzpositionen die in einem Alignment identisch sind. Sie wird meist in Prozent der Alignment Länge angegeben.

Der Begriff "Ähnlichkeit", wie er hierin verwendet wird, setzt dagegen die Definition einer Ähnlichkeitsmetrik voraus, also eines Maßes dafür, als wie ähnlich man beispielsweise ein Valin zu einem Threonin oder zu einem Leucin annehmen möchte.

Der Begriff "Homologie", wie er hierin verwendet wird, bedeutet wiederum evolutionäre Verwandtschaft. Zwei homologe Proteine haben sich aus einer gemeinsamen Vorläufersequenz entwickelt. Der Begriff hat nicht unbedingt etwas mit Identität oder Ähnlichkeit zu tun, abgesehen davon, dass homologe Sequenzen meist ähnlicher sind (oder in einem Alignment mehr identische Positionen besitzen) als nichthomologe Sequenzen.

Der Ausdruck "RPI" wie er hierin verwendet wird, steht für Ribose-5-phosphat-Isomerase, die D-Ribose-5-phosphat in D-Ribulose-5-phosphat umwandelt.

Der Ausdruck "vollständige RPI" wie er hierin verwendet wird, beschreibt die RPI, die von der vollständigen kodierenden Region einer Transkriptiopseinheit kodiert wird, beginnend mit dem ATG-Startcodon und umfassend alle informationstragenden Exonbereiche des im Herkunftsorganismus vorliegenden, für RPI kodierenden Gens, sowie die für eine korrekte Termination der Transkription nötigen Signale.

Der Ausdruck "biologische Aktivität einer RPI" wie er hierin verwendet wird, bezieht sich auf die Fähigkeit eines Polypeptids, die vorstehend beschriebene Reaktion, d.h. die Umwandlung von D-Ribose-5-phosphat in D-Ribulose-5-phosphat zu katalysieren.

Der Ausdruck "aktives Fragment" wie er hierin verwendet wird, beschreibt nicht mehr-vollständige Nukleinsäuren kodierend für RPI, die aber noch für Polypeptide mit der biologischen Aktivität einer RPI kodieren, und die eine für die RPI charakteristische Reaktion wie vorne beschrieben katalysieren können. Solche Fragmente sind kürzer als die oben beschriebenen vollständigen, für die RPI kodierenden Nukleinsäuren. Dabei können sowohl an den 3'- und/oder 5'-Enden der Sequenz Nukleinsäuren entfernt worden sein, es können aber auch Teile der Sequenz deletiert; d.h. entfernt worden sein, die die biologische Aktivität der RPI nicht entscheidend beeinträchtigen. Eine geringere oder gegebenenfalls auch eine erhöhte Aktivität, die aber noch die Charakterisierung bzw. Verwendung des resultierenden RPI Fragments gestattet, wird dabei als ausreichend im Sinne des hier verwendeten Ausdrucks verstanden. Der Ausdruck "aktives Fragment" kann sich ebenso auf die Aminosäuresequenz der RPI beziehen und gilt dann analog den obigen Ausführungen für solche Polypeptide, die im Vergleich zur oben definierten vollständigen Sequenz bestimmte Teile nicht mehr enthalten, wobei die biologische Aktivität des Enzyms jedoch nicht entscheidend beeinträchtigt ist.

Der Ausdruck "Gen", wie er hierin verwendet wird, ist die Bezeichnung für einen Abschnitt aus dem Genom einer Zelle, die für die Synthese einer Polypeptid-Kette verantwortlich ist.

Der Ausdruck "cDNA" wie er hierin verwendet wird, beschreibt komplementäre, von einer mRNA durch reverse Transkription erhaltene DNA. Sie enthält nur die den Exons der genomischen DNA entsprechenden Sequenzen. Nach cDNA-Sequenzierung lässt sich gegebenenfalls die Aminosäuresequenz des davon kodierten Proteins ableiten. Nach Einführung einer cDNA in eine Zelle können von dem jeweiligen davon kodierten Protein große Mengen synthetisiert werden.

Der Ausdruck "hybridisieren", wie er hierin verwendet wird, beschreibt den Vorgang, bei welchem ein einzelsträngiges Nukleinsäuremolekül mit einem komplementären Strang eine Basenpaarung eingeht. Auf diese Weise können z.B. ausgehend von der hierin genannten oder ableitbaren Sequenzinformation beispielsweise DNA-Fragmente aus anderen phytopathogenen Pilzen als *Ustilago maydis* isoliert werden, welche für RPI kodieren, welche dieselben oder ähnliche Eigenschaften einer der erfindungsgemäße RPI aufweisen.

Hybridisierungsbedingungen werden nach folgender Formel näherungsweise berechnet:

Die Schmelztemperatur Tₘ:
Tₘ = 81,5°C + 16,6 {log[c(Na⁺)]} + 0,41(% G + C) - (500/n), nach Lottspeich, F., Zorbas H. (Hrsg.). (1998): Bioanalytik. Spektrum Akademischer Verlag, Heidelberg, Berlin.

Dabei ist c die Konzentration und n die Länge des hybridisierenden Sequenzabschnitts in Basenpaaren. Für eine Sequenz >100 bp entfällt der Ausdruck 500/n. Mit höchster Stringenz wird bei einer Temperatur 5-15°C unterhalb Tm und einer Ionenstärke von 15 mM Na⁺ (entspricht 0.1 x SSC) gewaschen. Wird eine RNA-Probe zur Hybridisierung verwendet, so ist der Schmelzpunkt um 10-15°C höher.

### Bevorzugte Hybridisierungsbedingungen sind nachstehend angegeben:

Hybridisierungslösung: DIG Easy Hyb (Roche, ZZ) Hybridisierungstemperatur: 42°C bis 70°C, bevorzugt bei 42-65°C (DNA-DNA) oder 50°C (DNA-RNA). Im vorliegenden Fall besonders geeignete stringente Temperaturen für die Hybridisierung liegen zwischen 50 und 65°C, wobei eine Temperatur von 65°C eine insbesondere geeignete stringente Temperatur darstellt.
1. Waschschritt: 2 x SSC, 0,1 % SDS 2 x 5 min bei Raumtemperatur;
2. Waschschritt: 1 x SSC, 0,1 % SDS 2 x 15 min bei 50°C; bevorzugt 0,5 x SSC, 0,1 % SDS 2 x 15 min bei 65°C; besonders bevorzugt 0,2 x SSC, 2 x 15 min bei 68°C.

Der Grad der Identität der Nukleinsäuren oder Aminosäuren wird vorzugsweise bestimmt mit Hilfe des Programms NCBI BLASTN Version 2.0.4. (Altschul et al. (1997) "Gapped BLAST and PSI-BLAST: a new generation of protein database search programs", *Nucleic Acids Res.* 25:3389).

Der Ausdruck "Fungizid" bzw. "fungizid" wie er hierin verwendet wird, bezieht sich auf chemische Verbindungen, die zur Bekämpfung solcher Pilze geeignet sind, die Pflanzen, Pflanzenteile oder Pflanzenprodukte befallen und schädigen oder deren Ertrag bzw. Wert mindern. Zu den genannten Pflanzenteilen gehören beispielsweise Blätter,SamenundFrüchte (wiez.B.Beeren,Obst,Getreidekörner). Zu den genannten Pflanzenprodukten gehören aus den Pflanzen gewonnene Rohstoffe oder Substanzen wie beispielsweise Hölzer oder Fasern. Solche Pilze sind z.B. Plasmodiophoromycetes, Oomycetes, Chytridiomycetes, Zygomycetes, Ascomycetes, Basidiomycetes und Deuteromycetes, z.B. Pythium-Arten, wie beispielsweise *Pythium ultimum,* Phytophthora-Arten, wie beispielsweise *Phytophthora infestans,* Pseudoperonospora-Arten, wie beispielsweise *Pseudoperonospora humuli* oder *Pseudoperonospora cubensis,* Plasmopara-Arten, wie beispielsweise *Plasmopara viticola,* Bremia-Arten, wie beispielsweise *Bremia lactucae,* Peronospora-Arten, wie beispielsweise *Peronospora pisi* oder *P. brassicae,* Erysiphe-Arten, wie beispielsweise *Erysiphe graminis,* Sphaerotheca-Arten, wie beispielsweise *Sphaerotheca fuliginea,* Podosphaera-Arten, wie beispielsweise *Podosphaera leucotricha,* Venturia-Arten, wie beispielsweise *Venturia inaequalis,* Pyrenophora-Arten, wie beispielsweise *Pyrenophora teres* oder P. *graminea* (Konidienform: Drechslera, Syn: Helminthosporium), Cochliobolus-Arten, wie beispielsweise *Cochliobolus sativus* (Konidienform: Drechslera, Syn: Helminthosporium), Uromyces-Arten, wie beispielsweise *Uromyces appendiculatus,* Puccinia-Arten, wie beispielsweise *Puccinia recondita,* Sclerotinia-Arten, wie beispielsweise *Sclerotinia sclerotiorum,* Tilletia Arten, wie beispielsweise *Tilletia caries;* Ustilago-Arten, wie beispielsweise *Ustilago nuda* oder *Ustilago avenae,* Pellicularia-Arten, wie beispielsweise *Pellicularia sasakii,* Pyricularia-Arten, wie beispielsweise *Pyricularia oryzae,* Fusarium-Arten, wie beispielsweise *Fusarium culmorum,* Botrytis-Arten wie z.B. *Botrytis cinerea,* Septoria-Arten, wie beispielsweise *Septoria nodorum,* Leptosphaeria-Arten, wie beispielsweise *Leptosphaeria nodorum,* Cercospora-Arten, wie beispielsweise *Cercospora canescens,* Alternaria-Arten, wie beispielsweise *Alternaria brassicae* oder Pseudocercosporella-Arten, wie beispielsweise *Pseudocercosporella herpotrichoides.* Von besonderem Interesse sind z.B. auch *Magnaporthe grisea, Cochliobulus heterostrophus, Nectria hematococcus* und Phytophtora Spezies.

Der Ausdruck "Fungizid" bzw. "fungizid" bezieht sich jedoch ebenso auf chemische Verbindungen, die zur Bekämpfung human- oder tierpathogener Pilze geeignet sind, also auch auf Antimykotika. Dazu gehören z.B. die folgenden humanpathogenen Pilze, die bestimmte Krankheitsbilder hervorrufen können: Dermatophyten, wie z.B. Trichophyton spec., Microsporum spec., *Epidermophyton floccosum* oder *Keratomyces ajelloi,* die z.B. Fußmykosen (Tinea pedis) hervorrufen, Hefen, wie z.B. *Candida albicans,* Soor-Ösophagitis und Dermatitis hervorruft, *Candida glabrata, Candida krusei* oder *Cryptococcus neoformans,* die z.B. pulmonale Cryptococcose und auch Torulose hervorrufen können, Schimmelpilze, wie z.B., *Aspergillus fumigatus, A. flavus, A. niger,* die z.B. bronchopulmonale Aspergillose oder Pilzsepsis hervorrufen, Mucor spec., Absidia spec., oder Rhizopus spec., die z.B. Zygomykosen (intravasale Mykosen) hervorrufen, *Rhinosporidium seeberi,* der z.B. chronische granulomatöse Pharyngitis und Tracheitis hervorruft, *Madurella myzetomatis,* der z.B. subkutane Myzetome hervorruft, *Histoplasma capsulatum,* der z.B. retikuloendotheliale Zytomykose und M. Darling hervorruft, *Coccidioides immitis,* der z.B. pulmonale Coccidioidomykose und Sepsis hervorruft, *Paracoccidioides brasiliensis,* der z.B. brasilianische Blastomykose hervorruft, *Blastomyces dermatitidis,* der z.B. Gilchrist-Krankheit und nordamerikanische Blastomykose hervorruft, *Loboa loboi*, der z.B. Keloid-Blastomykose und Lobo's Krankheit hervorruft, und *Sporothrix schenckii,* der z.B. Sporotrichose (granulomatöse Hautmykose) hervorruft.

Der Ausdruck "heterologer Promotor", wie er hierin verwendet wird, bezieht sich auf einen Promotor, der andere Eigenschaften als derjenige Promotor aufweist, der im Ursprungsorganismus die Expression des betreffenden Gens kontrolliert.

Der Ausdruck "Kompetitor" wie er hierin verwendet wird, bezieht sich auf die Eigenschaft der Verbindungen, mit anderen, gegebenenfalls zu identifizierenden Verbindungen um die Bindung an der RPI zu kompetitieren und diese vom Enzym zu verdrängen bzw. von dieser verdrängt zu werden.

Der Ausdruck "Agonist", wie er hierin verwendet wird, bezieht sich auf ein Molekül, das die Aktivität der RPI beschleunigt oder verstärkt.

Der Ausdruck "Antagonist", wie er hierin verwendet wird, bezieht sich auf ein Molekül, das die Aktivität der RPI verlangsamt oder verhindert.

Der Ausdruck "Modulator", wie er hierin verwendet wird, stellt den Oberbegriff zu Agonist bzw. Antagonist dar. Modulatoren können kleine organisch-chemische Moleküle, Peptide oder Antikörper sein, die an die erfindungsgemäßen Polypeptide binden bzw. die deren Aktivität beeinflussen. Weiterhin können Modulatoren kleine organisch-chemische Moleküle, Peptide oder Antikörper sein, die an ein Molekül binden, welches wiederum an die erfindungsgemäßen Polypeptide bindet, und dadurch deren biologische Aktivität beeinflusst. Modulatoren können natürliche Substrate und Liganden darstellen oder strukturelle oder funktionelle Mimetika davon. Bevorzugt handelt es sich beim Ausdruck "Modulator", wie er hierin verwendet wird, jedoch um solche Moleküle, die nicht die natürlichen Substrate bzw. Liganden darstellen.

Der Begriff "Kandidatenverbindung", wie er hierin verwendet wird, bezieht sich auf eine chemische Verbindung, die als potentieller Modulator oder Inhibitor in einem Verfahren zur Identifizierung von Modulatoren bzw. Inhibitoren der RPI eingesetzt und auf ihre Fähigkeit zur Modulation bzw. Inhibition des Polypeptids geprüft wird. Die Kandidatenverbindung, die eine entsprechende Wirkung zeigt, wird dann als Inhibitor bzw. Modulator, bzw. als Agonist oder Antagonist der RPI weiterverwendet und gegebenenfalls nach einer weiteren Prüfung ihrer Fähigkeit Pilze zu schädigen oder zu töten als Fungizid verwendet.

Im Rahmen der vorliegenden Erfindung wird die Nukleinsäuresequenz kodierend für die RPI aus dem pflanzenpathogenen Pilz *U maydis* und das davon, kodierte Polypeptid zugänglich gemacht. Weiterhin wird ein Verfahren zur Verfügung gestellt, das geeignet ist, die Aktivität der RPI zu bestimmen sowie Inhibitoren des Enzyms auch in HTS- und UHTS-Verfahren zu identifizieren, wobei die identifizierten Verbindungen als Fungizide verwendet werden können. Im Rahmen der vorliegenden Erfindung wird weiter gezeigt, dass die Inhibtoren der RPI, insbesondere der RPI aus Pilzen, bevorzugt aus pflanzenpathogenen Pilzen, als Pflanzenschutzmittel verwendet werden können.

Der Brandpilz *Ustilago maydis,* ein Basidiomycet, befällt Maispflanzen. Die Krankheit kommt in allen Maisanbaugebieten vor, erreicht jedoch nur in trockenen Jahren eine größere Bedeutung. Typische Symptome sind die beulenartigen, faustgroßen Anschwellungen (Brandbeulen), die an allen oberirdischen Pflanzenteilen gebildet werden. Die Beulen sind zuerst von einer weiß-grauen, derben Haut überzogen. Beim Aufreißen der Haut wird eine schwarze, zunächst schmierige, später pulvrige Brandsporenmasse frei. Weitere Arten der Gattung Ustilago sind z. B. *U. nuda* (verursacht Gersten- und Weizenflugbrand), *U. nigra* (verursacht Gerstenschwarzbrand), *U. hordei* (verursacht Gerstenhartbrand) und *U. avenae* (verursacht Haferflugbrand).

In *Ustilago maydis* wurde nun ein mögliches *rpi*-Gen annotiert (Um38_8). Zur Validierung wurde in *Ustilago maydis* ein Knock-out von Um38_8 *(rpil)* durchgeführt. Der Knock-out von Um38_8 erwies sich als letal, es handelt sich bei dem Genprodukt RPI1 des Gens Um38_8 *(rpil)* also auch im pflanzenpathogenen Pilz *U. maydis* um ein essentielles Enzym.

Das *rpil*-Gen aus *Ustilago maydis* besitzt eine Größe von 1020 bp, es enthält kein Intron. UM 38_8 *(rpil)* codiert für ein Polypeptid von 339 Aminosäuren Länge mit einem Molekulargewicht von ca. 37000 Dalton. Für die heterologe Expression des *rpil*-Gens wurde das Gen mit genspezifischen Oligonucleotiden mittels PCR amplifiziert und in den Expressionsvektor pET21b kloniert, sodass das RPI1-Protein ausgehend vom Plasmid pRPI2 mit einem C-terminalen His₆-Tag exprimiert wird. Es konnte weiterhin gezeigt werden, dass das in *E.coli* heterolog exprimierte Protein die enzymatische Aktivität einer RPI besitzt.

Im Rahmen der vorliegenden Erfindung wurde weiter gefunden, dass die RPI zum Identifizieren von Substanzen in geeigneten Testverfahren verwendet werden kann, die die Aktivität des Enzyms beeinflussen, was bei verschiedenen theoretisch interessanten Targets nicht selbstverständlich gegeben ist. Neben einer RPI aus einem phytopathogenen Pilz, die durch ihre Aminosäuresequenz und die dafür kodierende Nukleinsäuresequenz charakterisiert wird, werden deshalb auch geeignete Testverfahren zum Identifizieren von Modulatoren des Enzyms zur Verfügung gestellt, die auch zur Verwendung in HTS-Verfahren geeignet sind.

Im Rahmen der vorliegenden Erfindung wurde weiter gefunden, dass die RPI *in vitro* tatsächlich durch Wirkstoffe inhibiert werden kann und auch ein mit diesen Wirkstoffen *in vivo* behandelter pilzlicher Organismus durch die Behandlung mit diesen Wirkstoffen geschädigt und abgetötet werden kann. Die Inhibitoren einer RPI aus pflanzenpathogenen Pilzen können also als Fungizide im Pflanzenschutz verwendet werden. In der vorliegenden Erfindung wird beispielsweise gezeigt, dass die Hemmung der RPI mit in einem oben genannten Testverfahren identifizierten Substanzen zum Absterben der behandelten Pilze in synthetischen Medien bzw. auf der Pflanze führt.

Wie bereits vorstehend geschildert wurde, war trotz der intensiven Forschung an RPI bislang unbekannt, dass die RPI in pflanzenpathogenen Pilzen ein Zielprotein (ein so genanntes "Target") füngizid wirksamer Substanzen sein kann. Damit wird in der vorliegenden Erfindung zum ersten Mal gezeigt, dass die RPI ein insbesondere für phytopathogene Pilze wichtiges Enzym darstellt und deshalb in besonderem Maße dazu geeignet ist, als Zielprotein für die Suche nach weiteren und verbesserten fungizid wirksamen Wirkstoffen verwendet zu werden.

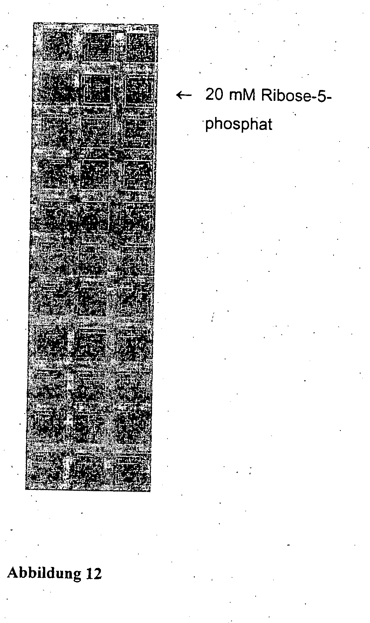

RPIs teilen sich mehrere homologe Bereiche (vgl. Abb. 11). Mit Hilfe eines Sequenzvergleichs konnten im Rahmen der vorliegenden Erfindung mehrere Konsensusbereiche identifiziert werden, die dazu dienen können, RPIs auch in anderen Pilzen zu identifizieren und zu charakterisieren.

Die spezifischen Konsensussequenzen, die zur Identifizierung bzw. Zuordnung weiterer erfindungsgemäßer Polypetide genutzt werden können, sind
(a) -(I/V)GIGSGSTV-,
(b) -(P)TG(F/D)QSX₂LI-,
(c) -(I/V)D(I/V)X₂DGADE(I/V)DX₂LX₂IKGG-,
(d) -EK(V/L)X₄AX₂F(I/V)XVADX(R/S)K-,
(e) -WX₂G(I/V)PIEVXP-,
(f) -AKAGP(I/V)VTDNXNFX(I/V/L)D-,
(g) -IKXLXGVXEXGLF-, und
(h) -AYFGNXDG-,

insbesondere die unter (b), (d), (g) und (h) genannten Sequenzen, wobei die in Klammern angegebenen Aminosäuren alternativ vorliegen können und wobei der Buchstabe X eine Position beschreibt, an der jede Aminosäure akzeptiert wird. Die Anzahl der möglichen Aminosäuren X wird durch eine tiefgestellte Zahl angegeben.

Die genannten Konsensussequenzen sind typisch für Polypeptide mit der biologischen Aktivität einer RPI.

Gegenstand der vorliegenden Erfindung sind deshalb auch Polypeptide aus pflanzenpathogenen Pilzen mit der biologischen Aktivität einer RPI, die eine, bevorzugt mehrere und insbesondere alle der vorstehend genannten Konsensussequenzen umfassen.

Gegenstand der Erfindung sind weiterhin auch Verfahren zum Identifizieren von Fungiziden, die auf Hemmtests basieren, welche mit einem Polypeptid mit der biologischen Aktivität einer RPI, die eine, mehrere oder alle der vorstehend genannten Konsensussequenzen umfasst, durchgeführt werden.

Aufgrund der vorstehenden Ergebnisse und der Homologien, die bei speziesspezifischen Nukleinsäuren kodierend für RPI vorliegen, kann auch eine RPI aus einem anderen Organismus, insbesondere auch aus anderen pflanzenpathogenen Pilzen gewonnen und in einem erfindungsgemäßen Verfahren verwendet werden, um die oben gestellte Aufgabe zu lösen, d.h. sie kann ebenfalls zum Identifizieren von Fungiziden verwendet werden. Es ist jedoch auch denkbar einen anderen Pilz, der nicht pflanzenpathogen ist, bzw. dessen RPI oder die dafür kodierende Sequenz zu verwenden, um fungizid wirkende Inhibitoren der RPI zu identifizieren, wie z.B. *S*. *cerevisiae.* Aufgrund der in der aus S. *cerevisiae* bekannten, für RPI kodierenden Nukleinsäuresequenz oder der in der vorliegenden Anmeldung angegebenen Sequenz gemäß SEQ ID NO: 1 können Oligonukleotidprimer abgeleitet werden und dadurch z.B. mittels PCR weitere für RPI kodierende Nukleinsäuren aus anderen pflanzenpathogenen Pilzen erhalten werden. Solche Nukleinsäuren und deren Verwendung im erfindungsgemäßen Verfahren werden als von der vorliegenden Erfindung umfasst betrachtet.

Gegenstand der vorliegenden Erfindung sind deshalb Nukleinsäuren, die für vollständige Polypeptide aus pflanzenpathogenen Pilzen mit der biologischen Aktivität einer RPI kodieren. Diese Nukleinsäuren weisen bevorzugt eine Identität von mindestens 70 %, mindestens 75 %, mindestens 80 %, mindestens 85 %, mindestens 90 %, mindestens 95 % und insbesondere mindestens 98 % über eine Länge von 60, 300, 600 oder 1200 Basenpaaren und bevorzugt über die Gesamtlänge der kodierenden Sequenz zueinander und insbesondere zur erfindungsgemäßen Sequenz gemäß SEQ ID NO:1 auf.

Gegenstand der vorliegenden Erfindung sind insbesondere Nukleinsäuren, die für eine RPI aus pflanzenpathogenen Basidiomyceten, bevorzugt aus der Gattung *Ustilago* kodieren.

Gegenstand der vorliegenden Erfindung sind ganz besonders bevorzugt Nukleinsäuren, die für die RPI aus *Ustilago maydis* kodieren.

Gegenstand der vorliegenden Erfindung sind insbesondere bevorzugt Nukleinsäuren aus *Ustilago maydis,* die für ein Polypeptid gemäß SEQ ID NO:2 oder aktive Fragmente davon kodieren. Insbesondere ist die Nukleinsäure gemäß SEQ ID NO: 1 Gegenstand der vorliegenden Erfindung.

Bei den erfindungsgemäßen Nukleinsäuren handelt es sich insbesondere um einzelsträngige oder doppelsträngige Desoxyribonukleinsäuren (DNA) oder Ribonukleinsäuren (RNA). Bevorzugte Ausführungsformen sind Fragmente genomischer DNA, die Introns enthalten können, und cDNAs.

Bevorzugt. handelt es sich bei den erfindungsgemäßen Nukleinsäuren um DNA-Fragmente, die der cDNA der erfindungsgemäßen Nukleinsäuren entsprechen.

Besonders bevorzugt umfassen die erfindungsgemäßen Nukleinsäuren eine Sequenz aus phytopathogenen Pilzen kodierend für ein Polypeptid mit der biologischen Aktivität einer RPI ausgewählt aus
(a) der Sequenz gemäß SEQ ID NO: 1,
(b) Sequenzen, die für ein Polypeptid kodieren, welches eine Aminosäuresequenz gemäß SEQ ID NO: 2 umfasst,
(c) Sequenzen, welche für ein Polypeptid kodieren, welches zumindest eine der vorstehend genannten Konsensussequenzen (a) bis (h) umfasst,
(d) zumindest 30 Basenpaare lange Teilsequenzen der unter a) bis c) definierten Sequenzen,
(e) Sequenzen, welche an die unter a) bis c) definierten Sequenzen bei einer Hybridisierungstemperatur von 42-65 °C hybridisieren,
(f) Sequenzen, welche eine zumindest 70 %ige, bevorzugt eine 80 %ige, besonders bevorzugt eine 90 %ige und ganz besonders bevorzugt eine 95 %ige Identität mit den unter a) bis c) definierten Sequenzen aufweisen,
(g) Sequenzen, welche zu den unter a) bis e) definierten Sequenzen komplementär sind, und
(h) Sequenzen, welche aufgrund der Degeneriertheit des genetischen Codes für dieselbe Aminosäuresequenz codieren wie die unter a) bis c) definierten Sequenzen.

Die erfindungsgemäßen Nukleinsäuren können auf die übliche Weise hergestellt werden. Beispielsweise können die Nukleinsäuremoleküle vollständig chemisch synthetisiert werden. Man kann auch kurze Stücke der erfindungsgemäßen Nukleinsäuren chemisch synthetisieren und solche Oligonukleotide radioaktiv oder mit einem Fluoreszenzfarbstoff markieren. Diese markierten Oligonukleotide können auch verwendet werden, um von mRNA z.B. aus phytopathogenen Pilzen hergestellte cDNA-Banken zu durchsuchen. Klone, an die die markierten Oligonukleotide hybridisieren, werden zur Isolierung der betreffenden DNA-Fragmente ausgewählt. Nach der Charakterisierung der isolierten DNA erhält man auf einfache Weise die erfindungsgemäßen Nukleinsäuren.

Die erfindungsgemäßen Nukleinsäuren können auch mittels PCR-Verfahren unter Verwendung chemisch synthetisierter Oligonukleotide hergestellt werden.

Der Ausdruck "Oligonukleotid(e)", wie er hierin verwendet wird, bezieht sich auf DNA-Moleküle, die aus 10 bis 50 Nukleotiden, vorzugsweise 15 bis 30 Nukleotiden bestehen. Sie werden chemisch synthetisiert und können als Sonden für Hybridisierungsversuche oder als Primer für PCR (Polymerase Chain Reaction) verwendet werden.

Zur Herstellung der erfindungsgemäßen Polypeptide, insbesondere des von der Nukleinsäuresequenz gemäß SEQ ID NO: 1 kodierten Polypeptids, können außerdem Wirtszellen, die mindestens eine der erfindungsgemäßen Nukleinsäuren enthalten, unter geeigneten Bedingungen kultiviert werden. Die gewünschten Polypeptide können danach auf übliche Weise aus den Zellen oder dem Kulturmedium isoliert werden. Die Polypeptide können auch in *in* vitro-Systemen hergestellt werden.

Zur Herstellung der erfindungsgemäßen RPI aus *Ustilago maydis* kann das Gen z.B. rekombinant in *Escherichia coli* exprimiert und aus *E. coli* Zellen eine Enzympräparation hergestellt werden (vgl. Beispiel 1).

Wie bereits vorstehend für die Nukleinsäuren ausgeführt, ist die vorliegende Erfindung nicht nur auf die Sequenz gemäß SEQ ID NO: 2 beschränkt. Die Ergebnisse, die im Rahmen der vorliegenden Erfindung hier zum ersten Mal gezeigt werden, gelten ebenso auch für andere Polypeptide aus pflanzenpathogenen Pilzen mit der biologischen Aktivität einer RPI. So kann auch eine homologe RPI aus anderen Pilzspezies, wie vorstehend im Rahmen der Definition zum Begriff "Fungizid" ausgeführt, erhalten und in erfindungsgemäßen Verfahren verwendet werden. Polypeptide aus pflanzenpathogenen Pilzen mit der biologischen Aktivität einer RPI werden deshalb als vom Gegenstand der vorliegenden Erfindung umfasst betrachtet.

Solche zur RPI aus *Ustilago maydis,* insbesondere zum Polypeptid gemäß SEQ ID NO: 2 homologen Polypeptide, die zum Identifizieren von fungiziden Wirkstoffen verwendet werden können, müssen keine vollständige pilzliche RPI darstellen, sondern können auch nur Fragmente davon sein, solange sie zumindest noch die biologische Aktivität der vollständigen RPI aufweisen. Polypeptide, die eine gleichartige biologische Aktivität wie eine RPI mit einer Aminosäuresequenz gemäß SEQ ID NO: 2 ausüben, werden noch als erfindungsgemäß betrachtet. Als erfindungsgemäß werden vor allem noch solche Polypeptide betrachtet, die einer RPI beispielsweise der vorstehend unter Definitionen zum Begriff "Fungizid" aufgeführten pflanzenpathogenen Pilze entsprechen oder Fragmenten davon, die noch deren biologische Aktivität haben.

Bevorzugt umfassen die erfindungsgemäßen Polypeptide damit eine Aminosäuresequenz aus pflanzenpathogenen Pilzen, ausgewählt aus:
(a) der Sequenz gemäß SEQ ID NO: 2,
(b) Sequenzen umfassend zumindest eine der vorstehend genannten Konsensussequenzen (a) bis (h),
(c) zumindest 15 Aminosäuren lange Teilsequenzen der unter (a) und (b) definierten Sequenzen,
(d) Sequenzen, welche eine zumindest 60 %ige, bevorzugt eine 70 %ige, besonders bevorzugt eine zumindest 80 %ige und ganz besonders bevorzugt eine 90 %ige Identität mit der unter (a) definierten Sequenz haben, und
(e) Sequenzen, welche die gleiche biologische Aktivität aufweisen wie die unter (a) definierte Sequenz.

Der Ausdruck "Polypeptide", wie er hierin verwendet wird, bezieht sich sowohl auf kurze Aminosäureketten, die gewöhnlich als Peptide, Oligopeptide oder Oligomere bezeichnet werden, als auch auf längere Aminosäureketten, die gewöhnlich als Proteine bezeichnet werden. Er umfasst Aminosäureketten, die entweder durch natürliche Prozesse, wie posttranslationale Prozessierung, oder durch chemische Verfahren, die Stand der Technik sind, modifiziert sein können. Solche Modifikationen können an verschiedenen Stellen und mehrfach in einem Polypeptid vorkommen, wie beispielsweise am Peptid-Rückgrat, an der Aminosäure-Seitenkette, am Aminound/oder am Carboxy-Terminus. Sie umfassen beispielsweise Acetylierungen, Acylierungen, ADP-Ribosylierungen, Amidierungen, kovalente Verknüpfungen mit Flavinen, Häm-Anteilen, Nukleotiden oder Nukleotid-Derivaten, Lipiden oder Lipid-Derivaten oder Phophatidylinositol, Cyclisierungen, Disulfidbrückenbildungen, Demethylierungen, Cystin-Bildungen, Formylierungen, gamma-Carboxylierungen, Gly-cosylierungen, Hydroxylierungen, Iodierungen, Methylierungen, Myristoylierungen, Oxidationen, proteolytische Prozessierungen, Phosphorylierungen, Selenoylierungen und tRNA-vermittelte Additionen von Aminosäuren.

Die erfindungsgemäßen Polypeptide können in der Form "reifer" Proteine oder als Teile größerer Proteine, z.B. als Fusionsproteine, vorliegen. Weiterhin können sie Sezernierungs- oder "Leader"-Sequenzen, Pro-Sequenzen, Sequenzen, die eine einfache Reinigung ermöglichen, wie mehrfache Histidin-Reste, oder zusätzliche stabilisierende Aminosäuren aufweisen. Die erfindungsgemäßen Proteine können ebenfalls so vorliegen, wie sie natürlicherweise in ihrem Herkunftsorganismus vorliegen, aus dem sie zum Beispiel direkt gewonnen werden können.

Die erfindungsgemäßen Polypeptide können im Vergleich zu den entsprechenden Regionen von natürlich vorkommender RPI Deletionen oder Aminosäuresubstitutionen aufweisen, solange sie zumindest noch die biologische Aktivität einer vollständigen RPI zeigen. Konservative Substitutionen sind bevorzugt. Solche konservativen Substitutionen umfassen Variationen, wobei eine Aminosäure durch eine andere Aminosäure aus der folgenden Gruppe ersetzt wird:
1. Kleine aliphatische, nicht-polare oder wenig polare Reste: Ala, Ser, Thr, Pro und Gly;
2. Polare, negativ geladene Reste und deren Amide: Asp, Asn, Glu und Gln;
3. Polare, positiv geladene Reste: His, Arg und Lys;
4. Große aliphatische, nicht-polare Reste: Met, Leu, Ile, Val und Cys; und
5. Aromatische Reste: Phe, Tyr und Trp.

Die folgende Liste zeigt bevorzugte konservative Substitutionen:

| **Ursprünglicher Rest** | **Substitution** |
|---|---|
| Ala | Gly, Ser |
| Arg | Lys |
| Asn | Gln, His |
| Asp | Glu |
| Cys | Ser |
| Gln | Asn |
| Glu | Asp |
| Gly | Ala, Pro |
| His | Asn, Gln |
| Ile | Leu, Val |
| Leu | Ile, Val |
| Lys | Arg, Gln, Glu |
| Met | Leu, Tyr, Ile |
| Phe | Met, Leu, Tyr |
| Ser | Thr |
| Thr | Ser |
| Trp | Tyr |
| Tyr | Trp, Phe |
| Val | Ile, Leu |

Ein mögliches Reinigungsverfahren der RPI basiert auf präparativer Elektrophorese, FPLC, HPLC (z.B. unter Anwendung von Gelfiltrations-, Umkehrphasen- oder hydrophoben Säulen), Gelfiltration, differentieller Präzipitation, IonenaustauschChromatographie oder Affinitätschromatographie.

Ein schnelles Verfahren zum Isolieren der erfindungsgemäßen Polypeptide, die von Wirtszellen unter Verwendung einer erfindungsgemäß zu verwendenden Nukleinsäure synthetisiert werden, beginnt mit der Expression eines Fusionsproteins, wobei der Fusionspartner auf einfache Weise affinitätsgereinigt werden kann. Der Fusionspartner kann beispielsweise ein 6xHis-Tag sein. Das Fusionsprotein kann dann an einer Nickel-NTA-Affinitätssäule gereinigt werden. Der Fusionspartner kann durch partielle proteolytische Spaltung beispielsweise an Linkern zwischen dem Fusionspartner und dem zu reinigenden erfindungsgemäßen Polypeptid abgetrennt werden. Der Linker kann so gestaltet werden, dass er Ziel-Aminosäuren, wie Arginin- und Lysin-Reste einschließt, die Stellen für eine Spaltung durch Trypsin definieren. Um solche Linker zu erzeugen, können Standard-Klonierungsverfahren unter Verwendung von Oligonukleotiden angewendet werden.

Weitere mögliche Reinigungsverfahren basieren wiederum auf präparativer Elektrophorese, FPLC, HPLC (z.B. unter Anwendung von Gelfiltrations-, Umkehrphasenoder leicht hydrophoben Säulen), Gelfiltration, differentieller Präzipitation, Ionenaustausch-Chromatographie und Affinitätschromatographie.

Die Ausdrücke "Isolierung oder Reinigung", wie sie hierin verwendet werden, bedeuten, dass die erfindungsgemäßen Polypeptide von anderen Proteinen oder anderen Makromolekülen der Zelle oder des Gewebes abgetrennt werden. Vorzugsweise ist eine die erfindungsgemäßen Polypeptide enthaltende Zusammensetzung hinsichtlich des Proteingehalts gegenüber einer Präparation aus den Wirtszellen mindestens 10-fach und besonders bevorzugt mindestens 100-fach angereichert.

Die erfindungsgemäßen Polypeptide können auch ohne Fusionspartner mit Hilfe von Antikörpern, die an die Polypeptide binden, affinitätsgereinigt werden.

Gegenstand der vorliegenden Anmeldung ist damit ebenfalls ein Verfahren zum Herstellen des Polypeptids mit einer Sequenz gemäß SEQ ID NO: 2 oder dazu homologer Polypeptide aus pflanzenpathogenen Pilzen mit der Aktivität einer RPI, welches gekennzeichnet ist durch
(a) das Kultivieren einer Wirtszelle enthaltend zumindest eine exprimierbare Nukleinsäuresequenz kodierend für ein Polypeptid aus pflanzenpathogenen Pilzen mit der biologischen Aktivität einer RPI unter Bedingungen, die die Expression dieser Nukleinsäure gewährleisten, oder
(b) das Exprimieren einer exprimierbaren Nukleinsäuresequenz kodierend für ein Polypeptid aus pflanzenpathogenen Pilzen mit der biologischen Aktivität einer RPI in einem *in vitro*-System, und
(c) die Gewinnung des Polypeptids aus der Zelle, dem Kulturmedium oder dem *in vitro*-System.

Insbesondere ist Gegenstand der vorliegenden Erfindung ein Verfahren zum Herstellen des Polypeptids mit einer Sequenz gemäß SEQ ID NO: 2 oder dazu homologer Polypeptide mit der Aktivität einer RPI aus pflanzenpathogenen Pilzen, wobei
(a) eine Nukleinsäuresequenz umfassend alle für eine Expression notwendigen Sequenzabschnitte und kodierend für ein Polypeptid aus pflanzenpathogenen Pilzen mit der biologischen Aktivität einer RPI in geeignete Wirtszellen transformiert wird,
(b) die transformierten Zellen bei einer geeigneten Temperatur inkubiert werden,
(c) die Zellen nach einem für ein ausreichendes Wachstum der Zellen geeigneten Zeitraum induziert werden,
(d) die Zellen nach einem für eine ausreichende Expression der unter (a) genannten Nukleinsäure geeigneten Zeitraum geerntet werden, und gegebenenfalls
(e) das Polypeptid aus den geernteten Zellen isoliert und gereinigt wird.

Die so erhaltenen Zellen enthaltend das erfindungsgemäße Polypeptid oder das so erhaltene gereinigte Polypeptid sind geeignet, in Verfahren zum Identifizieren von Modulatoren bzw. Inhibitoren der RPI bzw. zum Identifizieren von Fungiziden verwendet zu werden.

Gegenstand der vorliegenden Erfindung ist auch die Verwendung von Polypeptiden, welche zumindest eine biologische Aktivität einer RPI ausüben, in Verfahren zum Identifizieren von Fungiziden. Insbesondere ist der Gegenstand der vorliegenden Erfindung die Verwendung einer RPI aus Pilzen, bevorzugt aus pflanzenpathogenen Pilzen und insbesondere einer Aminosäuresequenz ausgewählt aus
(a) der Sequenz gemäß SEQ ID NO: 2,
(b) Sequenzen umfassend zumindest eine der vorstehend genannten Konsensussequenzen (a) bis (h),
(c) Sequenzen, welche eine zumindest 60 %ige, bevorzugt eine 70 %ige, besonders bevorzugt eine zumindest 80 %ige und ganz besonders bevorzugt eine 90 %ige Identität mit der unter (a) definierten Sequenz haben, und
(d) Sequenzen, welche die gleiche biologische Aktivität aufweisen wie die unter (a) bis (c) definierten Sequenzen,

in Verfahren zum Identifizieren von Fungiziden.

Besonders bevorzugt ist die Verwendung von Polypeptiden aus pflanzenpathogenen Basidiomyceten, besonders aus der Gattung *Ustilago,* insbesondere aus *Ustilago maydis,* wobei hier das Polypeptid gemäß SEQ ID NO: 2 besonders bevorzugt ist, in Verfahren zum Identifizieren von Inhibitoren eines Polypeptids mit der Aktivität einer RPI, wobei die Inhibitoren der RPI als Fungizide verwendet werden können.

Fungizide Wirkstoffe, die mit Hilfe einer RPI aus verschiedenen Organismen, bevorzugt mit einer erfindungsgemäßen RPI gefunden werden, können also auch mit einer RPI aus anderen phytopathogenen oder auch human- oder tierpathogenen Pilzspezies interagieren, wobei die Interaktion mit den unterschiedlichen in diesen Pilzen vorkommenden RPI nicht immer gleich stark sein muss. Dies erklärt unter anderem die beobachtete Selektivität wirksamer Substanzen.

Wie bereits vorstehend erläutert, ermöglicht die Verwendung der erfindungsgemäßen Nukleinsäuren bzw. Polypeptide in einem geeigneten Verfahren das Auffinden von Verbindungen, die an die erfindungsgemäßen Polypeptide binden und/oder die Verbindung inhibieren. Diese können dann als Fungizide bei Pflanzen oder als Antimykotika bei Menschen und Tieren angewandt werden.

Gegenstand der vorliegenden Erfindung ist deshalb insbesondere ein Verfahren, dass zur Identifizierung von fungiziden Wirkstoffen geeignet ist, die an die erfindungsgemäßen Polypeptide binden und/oder deren biologische Aktivität modulieren, d.h. aktivieren oder inhibieren.

Gegenstand der vorliegenden Erfindung ist insbesondere ein Verfahren zum Identifizieren von Fungiziden durch Testen von potentiellen Inhibitoren bzw. Modulatoren der enzymatischen Aktivität der RPI (Kandidatenverbindungen) in einem D-Ribulose-5-phosphat-Isomerase (RPI) -Hemmtest.

Viele Testsysteme, die die Prüfung von Verbindungen und natürlichen Extrakten zum Ziel haben, sind bevorzugt auf hohe Durchsatzzahlen ausgerichtet, um die Zahl der untersuchten Substanzen in einem gegebenen Zeitraum zu maximieren. Testsysteme, die auf zellfreiem Arbeiten beruhen, brauchen gereinigtes oder semigereinigtes Protein. Sie sind geeignet für eine "erste" Prüfung, die in erster Linie darauf abzielt, einen möglichen Einfluss einer Substanz auf das Zielprotein zu detektieren. Ist eine solche erste Prüfung erfolgt und eine oder mehrere Verbindungen, Extrakte etc. gefunden, kann die Wirkung solcher Verbindungen im Labor noch gezielter untersucht werden. So kann in einem ersten Schritt die Inhibierung oder Aktivierung des erfindungsgemäßen Polypeptids *in vitro* noch einmal geprüft werden, um im Anschluss daran die Wirksamkeit der Verbindung am Zielorganismus, hier einem oder mehreren pflanzenpathogenen Pilzen, zu testen. Die Verbindung kann dann gegebenenfalls als Ausgangspunkt für die weitere Suche und Entwicklung von fungiziden Verbindungen verwendet werden, die auf der ursprünglichen Struktur basieren, jedoch z.B. hinsichtlich Wirksamkeit, Toxizität oder Selektivität optimiert sind.

Verfahren, die geeignet sind, Fungizide bzw. Aktivatoren oder Inhibitoren bzw. Agonisten oder Antagonisten der erfindungsgemäßen Polypeptide zu identifizieren, beruhen in aller Regel auf der Bestimmung der Aktivität bzw. der biologischen Funktionalität des Polypeptids. Dazu kommen prinzipiell sowohl auf ganzen Zellen beruhende Verfahren *(in vivo* Verfahren) in Frage, wie auch Verfahren, die auf der Verwendung des aus den Zellen isolierten Polypeptids beruhen, das in gereinigter oder teilweise gereinigter Form oder auch als Rohextrakt vorliegen kann. Diese zellfreien *in vitro* Verfahren können ebenso wie *in vivo* Verfahren im Labormaßstab, in bevorzugter Weise aber auch in HTS oder UHTS Verfahren genutzt werden.

Um Fungizide bzw. Modulatoren aufzufinden, kann z.B. ein synthetischer Reaktionsmix (z.B. Produkte der *in vitro* Transkription) oder ein zellulärer Bestandteil, wie eine Membran, ein Kompartiment oder irgendeine andere Präparation, die die erfindungsgemäßen Polypeptide enthält, zusammen mit einem gegebenenfalls markierten Substrat oder Liganden der Polypeptide in Gegenwart und Abwesenheit eines Kandidatenmoleküls, das ein Agonist oder Antagonist sein kann, inkubiert werden. Die Fähigkeit des Kandidatenmoleküls die Aktivität der erfindungsgemäßen Polypeptide zu erhöhen oder zu hemmen, wird z.B. erkennbar an einer erhöhten oder verringerten Bindung des gegebenenfalls markierten Liganden oder an einer erhöhten oder verringerten Umsetzung des gegebenenfalls markierten Substrates. Moleküle, die zu einer erhöhten Aktivität der erfindungsgemäßen Polypeptide führen, sind Agonisten. Moleküle, die die biologische Aktivität der erfindungsgemäßen Polypeptide hemmen, sind Antagonisten.

Die Detektion der biologischen Aktivität der erfindungsgemäßen Polypeptide kann durch ein so genanntes Reportersystem verbessert werden. Reportersysteme in dieser Hinsicht umfassen, sind aber nicht beschränkt auf colorimetrisch oder fluorometrisch nachweisbare Substrate, die in ein Produkt umgewandelt werden oder ein Reportergen, das auf Veränderungen der Aktivität oder der Expression der erfindungsgemäßen Polypeptide anspricht oder andere bekannte Bindungstests.

Ein weiteres Beispiel für ein Verfahren, mit welchem Modulatoren der erfindungsgemäßen Polypeptide aufgefunden werden können, ist ein Verdrängungstest, bei dem man unter dafür geeigneten Bedingungen die erfindungsgemäßen Polypeptide und einen potenziellen Modulator mit einem Molekül, das bekanntermaßen an die erfindungsgemäßen Polypeptide bindet, wie einem natürlichen Substrat oder Liganden oder einem Substrat- oder Liganden-Mimetikum zusammenbringt. Die erfindungsgemäßen Polypeptide selbst können markiert werden, z.B. fluorimetrisch oder colorimetrisch, so dass man die Anzahl der Polypeptide, die an einen Liganden gebunden sind oder die eine Umsetzung mitgemacht haben, exakt bestimmen kann. Ebenso kann jedoch die Bindung mittels des gegebenenfalls markierten Substrats, Liganden bzw. Substratanalogen verfolgt werden. Auf diese Weise lässt sich die Effektivität eines Agonisten oder Antagonisten ermessen.

Effekte wie Zelltoxizität werden in diesen *in vitro* Systemen in der Regel ignoriert. Die Testsysteme überprüfen dabei sowohl inhibierende bzw. suppressive Effekte der Substanzen, als auch stimulatorische Effekte. Die Effektivität einer Substanz kann durch konzentrationsabhängige Testreihen überprüft werden. Kontrollansätze ohne Testsubstanzen bzw. ohne Enzym können zur Bewertung der Effekte herangezogen werden. Deshalb folgt einem solchen *in vitro* Testsystem in aller Regel die Prüfung der fungiziden Eigenschaften der identifizierten Verbindungen, indem man die Verbindungen mit verschiedenen Pilzen in Kontakt bringt und beurteilt, ob und inwieweit der Pilz durch die Verbindung geschädigt oder abgetötet wird.

Ein Verfahren zum Identifizieren von Fungiziden beruht darauf, dass man.
a) eine RPI oder eine Wirtszelle enthaltend dieses Polypeptid mit einer chemischen Verbindung oder mit einem Gemisch von chemischen Verbindungen unter Bedingungen in Kontakt bringt, die die Interaktion einer chemischen Verbindung mit dem Polypeptid erlauben,
b) die Aktivität der RPI bei Abwesenheit einer chemischen Verbindung mit der Aktivität des erfindungsgemäßen Polypeptids bei Anwesenheit einer chemischen Verbindung oder eines Gemisches von chemischen Verbindungen vergleicht, und
c) die chemische Verbindung bestimmt, die die Aktivität der RPI moduliert bzw. inhibiert.

Besonders bevorzugt wird dabei diejenige Verbindung bestimmt, die die Aktivität des erfindungsgemäßen Polypeptids spezifisch inhibiert. Der Begriff "Aktivität", wie er hier verwendet wird, bezieht sich auf die biologische Aktivität der RPI.

Die enzymatische Aktivität bzw. die Ab- oder Zunahme der enzymatischen Aktivität der RPI bzw. die Inhibition dieser enzymatischen Aktivität wird bevorzugt mittels der Umsetzung des Substrats NAD⁺ zu NADH bestimmt. Dabei wird die geringere bzw. inhibierte Aktivität des erfindungsgemäßen Polypeptids letztlich anhand der fluoreszenzspektrometrischen Bestimmung der geringeren Entstehung des NADH verfolgt. Aufgrund der Lichtabsorption des Nicotinamidringes besitzt das reduzierte Nicotinamid-Coenzym NADH ein Absorptionsmaximum bei 340 nm. Die oxidierte Form NAD⁺ zeigt dagegen zwischen 300 und 400 nm keine Absorption. Die erfindungsgemäße enzymatische Reaktion, die aufgrund einer anschließenden Kopplung weiterer enzymatischer Reaktionen zu einer Reduktion des NAD⁺ und der Entstehung von NADH führt, kann deshalb anhand der Zunahme der Absorption z.B. bei 340 nm verfolgt werden. NADH fluoresziert zudem stärker als NAD⁺. Der Einfluss eines Inhibitors auf die Reaktion kann damit ebenfalls bestimmt werden, wobei die geringere bzw. inhibierte Aktivität der RPI anhand eines geringeren Anstiegs der Fluoreszenz bestimmt wird, die vom im geringeren Maße entstehenden NADH ausgeht.

Das Verfahren basiert auf der Kopplung der enzymatischen Aktivität der RPI, die zur Entstehung von D-Ribulose-5-phosphat führt, mit weiteren Reaktionen, die schließlich zur Umsetzung von NAD⁺ zu NADH führen. Das Testsystem beinhaltet dabei drei weitere Hilfsenzyme, Ribulose-5-phosphat Epimerase (RPE), Transketolase (TK) und Glycerinaldehyd-3-phosphat Dehydrogenase (GAPDH). Dabei laufen die folgenden enzymatischen, aneinander gekoppelten Reaktionen ab:

RPI
1. Schritt: D-Ribose-5-phosphat → D-Ribulose-5-phosphat
RPE
2. Schritt: D-Ribulose-5-phosphat → Xylulose-5-phosphat
TK
3. Schritt: D-Ribose-5-phosphat → Sedoheptulose-7-phosphat
+ Xylulose-5-phosphat + Glycerinaldehyd-3-phosphat
GAPDH
4. Schritt: Glycerinaldehyd-3-phosphat →3-Phosphoglycerat + NADH + H+
+ NAD+ + AsO43- + AsO43-

Das Gleichgewicht der GAPDH-Reaktion liegt fast vollständig auf der Seite von Glycerinaldehyd-3-phosphat und NAD⁺. NADH wird erst in nennenswertem Umfang gebildet, wenn Arsenat anstelle des natürlichen Substrates Phosphat eingesetzt wird, da das entstehende Arsenat-Analogon des 1,3-Bisphosphoglycerats instabil ist und zerfällt und somit das Gleichgewicht nach rechts verschoben wird.

Die gekoppelten Enzyme werden im Vergleich zur RPI vorzugsweise im Überschuss eingesetzt, um die Isolierung von Inhibitoren dieser gekoppelten Enzyme zu vermeiden. Die Bildung von NADH im Verlauf der Reaktion wird dann anhand der Fluoreszenz bei einer Anregungswellenlänge von 360 nm und einer Emissionswellenlänge von 465 nm verfolgt (vgl. Abb. 3). Die Fluoreszenzausbeute bei Anwesenheit einer chemischen Verbindung kann dann mit der Fluoreszenzausbeute'bei Abwesenheit einer chemischen Verbindung verglichen werden. Der Vergleich zeigt dann, ob bei Anwesenheit einer chemischen Verbindung die Zunahme der Fluoreszenz geringer oder gegebenenfalls höher ist als bei Abwesenheit der chemischen Verbindung, d.h. ob die besagte Verbindung eine inhibitorische oder möglicherweise auch aktivatorische Wirkung auf das getestete Polypeptid hat. Der Zeitraum, in dem die Zunahme der Fluoreszenz gemessen wird, kann dabei variiert werden. Es kann ein langsamer Anstieg der Fluoreszenz im Verlauf einer bis mehrerer Stunden beobachtet werden. Um einen möglichst hohen Read-out, das heißt ein genügend großes Signal, in einem sinnvollen Zeitfenster zu erhalten, müssen alle Parameter wie Temperatur und Konzentration der Einzelkomponenten angepasst werden. Dazu wird unter anderem der K_{M}-Wert des Substrats D-Ribose-5-phosphat bestimmt, wobei ein Wert von 6,3 mM ermittelt wurde (vgl. Abbildung 4). Die Menge des eingesetzten D-Ribose-5-phosphats kann variiert werden, wobei eine Konzentration von 3 bis 10 mM bevorzugt wird (vgl. Abb. 5). Auch die Menge des eingesetzten NAD⁺ kann variiert werden, wobei hier eine Konzentration von 7 bis 40 mM besonders bevorzugt wird (vgl. Abb. 6). Die Proteinmenge kann ebenfalls variiert werden (vgl. Abb. 7), wobei Mengen von mehr als 25 ng RPI nicht sinnvoll sind, da ansonsten zunehmend auch Inhibitoren der gekoppelten Enzyme gefunden würden.

In der Literatur (Jung et al., *Arch. Biochem. Biophys.* 373 (2000) 409-417) wird Phosphat als "schwacher" Inhibitor der Ribose-phosphat-Isomerase beschrieben, weshalb der Test in Gegenwart von unterschiedlichen Konzentrationen an Phosphat durchgeführt wurde, um den Einfluss des Phosphats auf die Reaktion zu bestimmen. Ein zusätzliches Problem tritt in dem gekoppelten Assay dadurch auf, dass Phosphat auch als Substrat der GAPDH dient und damit die Kopplungsreaktion beschleunigt. Messungen ergaben, dass erst sehr hohe Konzentrationen an Phosphat einen Hemmeffekt zeigen. Phosphat ist deshalb in diesem Test nicht als Kontrollhemmstoff verwendbar.

Die Temperatur kann in einem relativ großen Bereich variiert werden, wobei Temperaturen von 18 bis 37°C bevorzugt werden. Besonders bevorzugt wird das Verfahren bei 37°C oder bei Raumtemperatur durchgeführt (vgl. Abb. 8).

Die Messung kann auch in für HTS- oder UHTS-Assays gängigen Formaten erfolgen, z.B. in Mikrotiterplatten, in denen z.B. ein Gesamtvolumen von 5 bis 50 µl pro Ansatz bzw. pro Well vorgelegt wird und die einzelnen Komponenten in einer der vorstehend angegebenen Endkonzentrationen vorliegen. Dabei wird die zu testende, potentiell die Aktivität des Enzyms inhibierende oder aktivierende Verbindung (Kandidatenmolekül) z.B. in einer geeigneten Konzentration in DMSO vorgelegt. Dann wird die Lösung, die das Substrat Ribose-5-phosphat enthält, zugegeben. Durch anschließende Zugabe einer Lösung mit dem erfindungsgemäßen Polypeptid wird die Reaktion gestartet. Der Ansatz wird dann z.B. bis zu 2 oder 3 Stunden bei einer geeigneten Temperatur inkubiert und die Fluoreszenzzunahme bei einer Anregungswellenlänge von 360 nm und einer Emissionswellenlänge von 465 nm gemessen.

Eine weitere Messung erfolgt in einem entsprechenden Ansatz, jedoch ohne Zugabe eines Kandidatenmoleküls und ohne Zugabe von RPI (Negativkontrolle). Eine weitere Messung erfolgt wiederum bei Abwesenheit eines Kandidatenmoleküls, jedoch bei Anwesenheit von RPI (Positivkontrolle). Negativ- und Positivkontrolle ergeben damit die Vergleichswerte zu den Ansätzen bei Anwesenheit eines Kandidatenmoleküls.

Ein weiteres Verfahren zum Identifizieren von Fungiziden ist die Durchführung eines Hemmtests, der auf einem spektrophotometrischen Verfahren beruht. In diesem Verfahren wird die enzymatische Aktivität der RPI anhand der Zunahme der Absorption bei 290 nm bestimmt. Die Zunahme der Absorption ist auf die Bildung von. Ribulose-5-phosphat zurückzuführen. Die Hemmung der enzymatischen Aktivität der RPI durch eine chemische Verbindung ist an einem geringeren oder keinem Anstieg der Absorption im Vergleich zu einem Ansatz ohne Kandidatenverbindung zu erkennen (vgl. Beispiel 3 (A)).

Ein weiteres Verfahren beruht auf dem Nachweis der Ketogruppe der durch die enzymatische Aktivität der RPI entstehenden Ribulose mit einer Carbazol/Cystein/HCl-Lösung (vgl. Beispiel 3 (B) und Abbildung 12). Die Hemmung der enzymatischen Aktivität einer RPI durch eine Kandidatenverbindung kann in diesem Fall durch eine geringere Färbung im Vergleich zu einem Ansatz ohne Kandidatenverbindung erkannt werden.

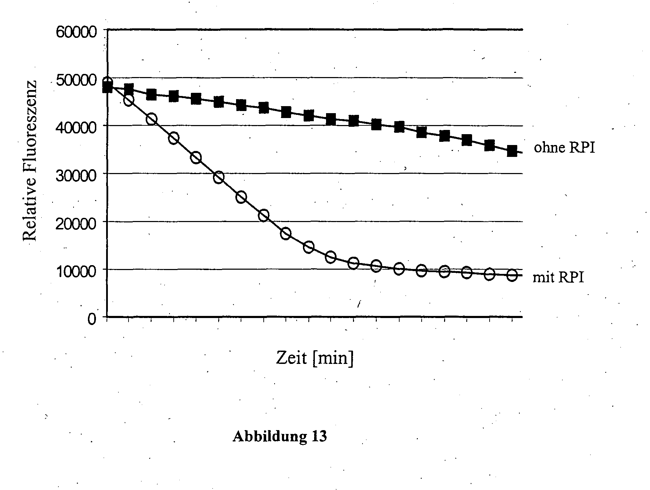

Ein weiterer gekoppelter Enzymtest basierend auf der Kopplung von RPI, Ribulose-5-phosphat-Kinase, Pyruvat-Kinase und Lactat-Dehydrogenase kann ebenfalls als ein Weg zur Identifizierung von Fungiziden dienen (vgl. Bsp. 3 (C) und Abb. 13).

Vorzugsweise wird in den erfindungsgemäßen Verfahren eine pilzliche RPI verwendet, insbesondere bevorzugt eine RPI aus pflanzenpathogenen Pilzen oder eine RPI aus Hefe, insbesondere aus *S. cerevisae.*

Mit Hilfe der vorstehend beispielhaft beschriebenen Verfahren konnten Verbindungen identifiziert werden, die eine RPI, insbesondere die erfindungsgemäßen RPI inhibieren, und die als Fungizide verwendet werden können.

Gegenstand der vorliegenden Erfindung ist deshalb insbesondere ein Verfahren zum Identifizieren von Fungiziden durch Testen einer Kandidatenverbindung in einem RPI-Hemmtest.

Vorzugsweise schließt sich an das erfindungsgemäße Verfahren ein weiterer Schritt an, in dem die fungizide Wirkung der identifizierten Verbindungen geprüft wird, indem Pilze mit der oder den Verbindungen in Kontakt gebracht und die Wirkung der Verbindungen auf diese Pilze geprüft wird.

Gegenstand der vorliegenden Erfindung ist insbesondere ein Verfahren wie vorstehend beschrieben, in dem die enzymatische Aktivität bzw. die Hemmung dieser Aktivität durch eine Kandidatenverbindung anhand der Enstehung von NADH in einer gekoppelten Reaktion mit RPE, TK und GAPDH oder mit Phosphoribulose-Kinase, Pyruvat-Kinase und Lactat-Dehydrogenase geprüft wird. Vorzugsweise wird dabei im ersten Fall das natürliche Substrat der GAPDH, Phosphat, durch Arsenat (AsO₄³⁻) ersetzt.

Gegenstand der vorliegenden Erfindung ist damit vorzugsweise ein Verfahren zum Identifizieren von Fungiziden, in dem man
a) eine RPI, bevorzugt eine pilzliche RPI, oder eine Wirtszelle enthaltend dieses Polypeptid mit einer chemischen Verbindung oder mit einem Gemisch von chemischen Verbindungen unter Bedingungen in Kontakt bringt, die die Interaktion einer chemischen Verbindung mit dem Polypeptid erlauben,
b) die Aktivität der RPI bei Abwesenheit einer chemischen Verbindung mit der Aktivität des erfindungsgemäßen Polypeptids bei Anwesenheit einer chemischen Verbindung oder eines Gemisches von chemischen Verbindungen vergleicht, indem man an die jeweilige enzymatische Reaktion der RPI weitere enzymatische Reaktionen koppelt, die zur Entstehung von NADH führen,
c) die chemische Verbindung bestimmt, die zu einer Verringerung der Menge des entstehenden NADH führt, und
d) gegebenenfalls im Anschluss daran die fungïzide Wirkung der Verbindung prüft, indem man sie mit einem pilzlichen Organismus in Kontakt bringt.

Es versteht sich von selbst, dass das Verfahren zum Identifizieren von Fungiziden nicht auf die vorstehend beschriebene Verfahren wie die gekoppelten Hemmtest beschränkt ist sondern das auch solche Aktivitäts- bzw. Hemmtests verwendet werden können, die geeignet sind, die enzymatische Aktivität einer RPI bzw. deren Hemmung bestimmen zu können. Dazu zählen auch bekannte Verfahren, die so modifiziert werden, dass zumindest noch der inhibitorische Effekt einer Kandidatenverbindung in diesem Verfahren erkannt werden kann, die Auswertung der Messergebnisse also möglich sein muss. Als statistischer Parameter kann z.B. das Signal-Hintergrund Verhältnis herangezogen werden. Eine geeignete Größe zur Bestimmung der Qualität eines Screeningverfahrens bzw. eines Hemmtests ist auch der z-Faktor. In den z-Faktor gehen neben der Differenz aus Signal und Hintergrund auch die Streuung aller Messwerte in die Berechnung mit ein. Der z-Faktor berechnet sich wie folgt: z-Faktor = 1-((3 x Standardabweichung posKo + 3 x Standardabweichung negKo) / (Mittelwert posKo - Mittelwert negKo)), wobei "posKo" für die Positivkontrolle steht und "negKo" für die Negativkontrolle (Zhang et al. (1999): A Simple Statistical Parameter for Use in Evaluation and Validation of High Throughput Screening Assays. *J. Biomol. Screen* 4(2):67-73).

Ein z-Faktor größer 0,7 gilt als exzellent, ein z-Faktor von 0,15-0,7 als gut, ein z-Faktor von 0,15 als noch ausreichend und ein z-Faktor von kleiner 0 als nicht mehr auswertbar.

Der z-Faktor im erfindungsgemäßen Verfahren liegt nach ca. 50 Minuten bei über 0,8 (vgl. Abb. 9).

Das erfindungsgemäße Verfahren ist dazu geeignet, die Aktivität einer RPI zu bestimmen und die Inhibition dieser Aktivität durch eine Kandidatenverbindung zu detektieren. Kandidatenverbindungen, die die Aktivität hemmen, können dann gezielt ausgewählt und gegebenenfalls weiter entwickelt werden.

In Tabelle I werden beispielhaft Verbindungen gezeigt, die mit einem erfindungsgemäßen Verfahren.als Inhibitoren der RPI identifiziert werden konnten.

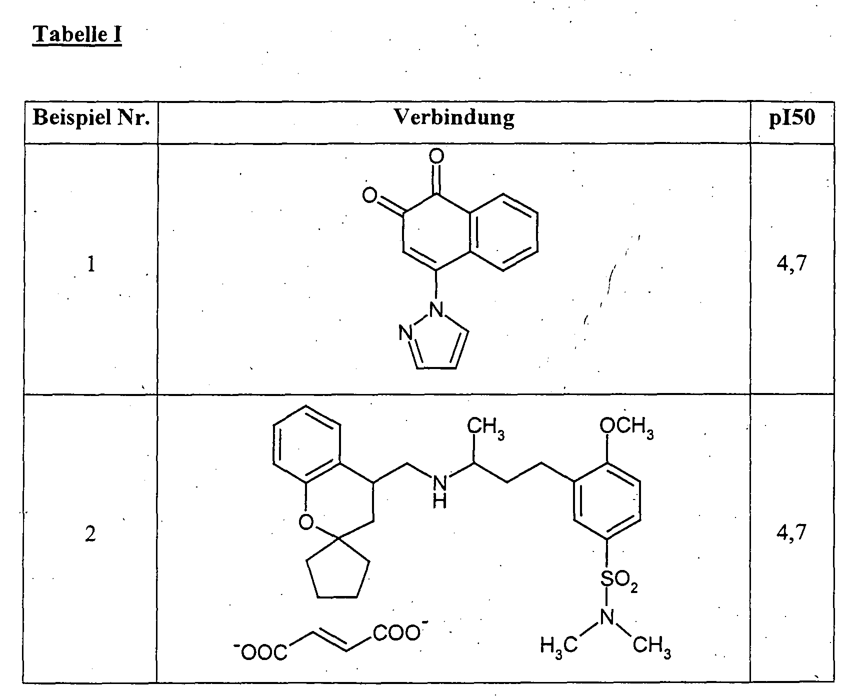

Der in Tabelle I angegebene pI50-Wert ist der negative dekadische Logarithmus des so genannten IC50-Werts, der die molare Konzentration einer Substanz angibt, die zur 50 %igen Hemmung eines Enzyms führt.

Ein pI50-Wert von 8 entspricht z.B. einer halbmaximalen Hemmung eines Enzyms bei einer Konzentration von 10 nM.

Im Rahmen der vorliegenden Erfindung konnte weiter gezeigt werden, dass die mit Hilfe eines erfindungsgemäßen Verfahrens identifizierten Inhibitoren. einer RPI geeignet sind, Pilze zu schädigen oder zu töten.

Dazu wurde z.B. in die Kavitäten von Mikrotiterplatten eine Lösung des zu prüfenden Wirkstoffs pipettiert. Nachdem das Lösungsmittel abgedampft war, wurde zu jeder Kavität Medium hinzugefügt. Das Medium wurde vorher mit einer geeigneten Konzentration von Sporen bzw. Mycel des zu prüfenden Pilzes versetzt. Die resultierenden Konzentrationen des Wirkstoffes betragen z.B. 0,1, 1, 10 und 100 ppm.

Die Platten wurden anschließend auf einem Schüttler bei einer Temperatur von 22°C inkubiert, bis in der unbehandelten Kontrolle ein ausreichendes Wachstum feststellbar war.

Die Auswertung erfolgte photometrisch bei einer Wellenlänge von 620 nm. Aus den Messdaten der verschiedenen Konzentrationen wurde die Wirkstoffdosis, die zu einer 50 %igen Hemmung des Pilzwachstums gegenüber der unbehandelten Kontrolle führt (ED₅₀) berechnet.

In Tabelle II sind beispielhaft Ergebnisse eines solchen Tests als ED₅₀-Werte für die in einem erfindungsgemäßen Verfahren gefundenen Verbindungen (vgl. Tabelle I) wiedergegeben.

**Tabelle II**

| **Verbindung (Beispiel-Nr.)** | **Organismus** | **ED**_{**50**}**[ppm]** |
|---|---|---|
| 1 | *Botrytis cinerea* | 9,68 |
| 1 | *Pyricularia oryzae* | 0,1 |
| 1 | *Phytophthora cryptogea* | 12,47 |
| 1 | *Ustilago avenae* | 22,07 |
| 2 | *Botrytis cinerea* | 9,68 |
| 2 | *Pyricularia* oryzae | 0,1 |
| 2 | *Phythophthora cryptogea* | 12,47 |
| 2 | *Ustilago avenae* | 22,07 |

Im Falle von *Erysiphe graminis* wurde weiterhin beispielhaft die Induktion einer Resistenz bei einer Pflanze gegen diesen Pilz getestet. Dafür findet 4 Tage nach der Behandlung der Pflanze mit der zu prüfenden Substanz eine Inokulation der Pflanze mit dem Erreger statt. Die Wirkung der Substanz wird als Wirkungsgrad in % angegeben. Es werden dabei Werte zwischen 0 und 100 errechnet, wobei ein Wirkungsgrad von 100 das Maximum an Wirkung bedeutet und 0 keine Wirkung. In diesem Versuch konnte mit der Verbindung gemäß Beispiel Nr. 2 (vgl. Tabelle I) eine Resistenzinduktion bei *Hordeum vulgaris* von 50 % festgestellt werden.

Gegenstand der vorliegenden Erfindung sind deshalb auch Inhibitoren der RPI, insbesondere der zur Inhibition von pflanzenpathogenen Pilzen geeigneten Verbindungen oder Extrakte, die mit Hilfe eines der in der vorliegenden Anmeldung beschriebenen Verfahrens zum Identifizieren von Inhibitoren der RPI gefunden werden.

Die vorliegende Erfindung bezieht sich auch auf Fungizide, die mit Hilfe eines erfindungsgemäßen Verfahrens identifiziert wurden.

Verbindungen, die mit Hilfe eines erfindungsgemäßen Verfahrens identifiziert werden, und die aufgrund der Inhibition der pilzlichen RPI eine fungizide Wirkung aufweisen, können dann zur Herstellung von fungiziden Mitteln verwendet werden.

Die identifzierten Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln. und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromatén oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthälocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Beim Einsatz der erfindungsgemäßen Verbindungen als Fungizide können die Aufwandmengen je nach Applikation innerhalb größerer Bereiche variiert werden.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermebrungsmaterial, insbesondere bei Samen, weiterhin durch ein- oder mehrschichtiges Umhüllen.

Die folgenden Beispiele sollen die verschiedenen Aspekte der vorliegenden Erfindung illustrieren und sind nicht limitierend auszulegen.

### Beispiele

### Beispiel 1

### Knock-out des rpi-Gens aus Ustilago maydis

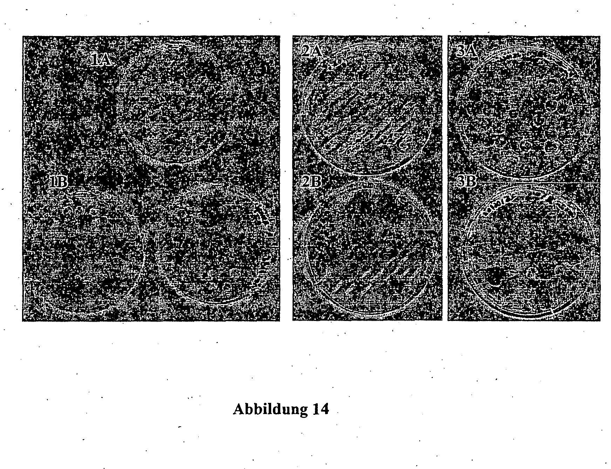

Um einen U maydis-rpi-knock-out-Stamm herzustellen, wurden genomische Flankenbereiche im 5'- und 3'-Bereich des rpi-Gens von ca. 1,4 kbp Länge durch eine PCR-Reaktion amplifiziert. Nach dem Schneiden der Flanken mit *SfiI* wurden diese zur Konstruktion; einer knock-out-Kassette mit einer Hygromycin-Resistenz-Kassette ligiert. Die so erhaltene knock-out-Kassette wurde mit "nested" angeordneten Primern in einer PCR amplifiziert. Nach Isolierung und Reinigung des PCR-Produktes wurde dieses in den diploiden *U. maydis* Stamm FBD11 transformiert. Die nach Selektion auf Hygromycin erhaltenen Transformanden wurden vereinzelt. Die knock-out Stämme wurden durch PCR-Analyse und Southern-Blot Analyse überprüft. Mit zwei der erhaltenen Stämme wurden Maispflanzen infiziert. Nach Keimen der erhaltenen Sporen wurden diese auf Hygromycin-Resistenz und das Vorhandensein des Wildtyp-Gens überprüft (Abbildung 14). Es könnten keine Sporen mit deletiertem *rpi-Gen* isoliert werden.

### Beispiel 2

### Klonierung, Expression und Reinigung von rpil bzw. RPI1 aus Ustilago maydis

Zur Expression der RPI1 wurde das Plasmid pRPI2 in *E.coli* BL21(DE3) transformiert. Als Vorkultur wurden 5 ml Selektionsmedium (dYT mit 100 µg/ml Ampicillin) mit einer Einzelkolonie angeimpft und bei 37°C über Nacht im Schüttler inkubiert. Die Hauptkultur (dYT mit 100 µg/ml Ampicillin) wurde 1:80 angeimpft und bei 37°C unter Schütteln inkubiert, nach Erreichen einer OD₆₀₀ von 0,7 wurde die Kultur 1 h bei 18°C inkubiert und anschließend durch Zugabe von 1 mM IPTG (Endkonzentration) induziert. Nach einer Inkubationszeit von 21 h bei 18°C wurden die Zellen geerntet und anschließend bei -20°C eingefroren. Die Zellen können auf diese Weise mehrere Monate bei -20°C ohne Aktivitätsverlust gelagert werden. 2,65 g Zellen wurden in 5 ml Bindepuffer (50 mM Kaliumphosphat-Puffer, pH 8,0; 10 % (v/v) Glycerin, 300 mM.NaCl) resuspendiert, 750 µl Lysozym (10 mg/ml) und 10 µl DNaseI (1mg/ml) wurden zugesetzt und 1h auf Eis inkubiert. Der Aufschluss erfolgte durch 3 Zyklen "Freeze and Thaw". Nach einer Zentrifugation bei 20000 rpm (JA20) und 4°C für 15 Minuten wurde der Überstand auf eine Ni-NTA-Säule (Volumen: 1 ml), die mit Bindepuffer äquilibriert war, aufgetragen. Die Säule wurde mit 10 ml Bindepuffer gewaschen, die Elution erfolgte in zwei Imidazol-Stufen 40 mM (4 ml) und 200 mM (2 ml), wobei die RPI1 in der 200mM-Stufe eluierte. Anschließend wurde die Proteinfraktion über eine PD10-Säule in Lagerungspuffer (50 mM Tris/HCl, pH8,0; 40 mM KCl, 10 mM MgCl₂) überführt. Aus 200 ml-*E. coli*-Kultur können ca. 3 mg lösliche RPI isoliert werden.

Der Proteinlösung wurde Glycerin (Endkonzentration 25 %(v/v)) zugesetzt, und diese dann bei -20°C eingefroren. Das auf diese Weise isolierte Enzym ist bei -20°C mehrere Monate ohne Aktivitätsverlust lagerbar.

### Beispiel 3

### RPI-Hemmtests zum Auffinden von Modulatoren

### A) Spektrophotometrisches Verfahren

Hierbei wird die enzymatische Aktivität der Ribose-phosphat-Isomerase anhand der Zunahme der Absorption bei 290 nm aufgrund der Bildung von Ribulose-5-phosphat aus Ribose-5-phosphat bestimmt. Dieser Test kann ebenso zur K_{M}-Wert- und zur Aktivitäts-Bestimmung einer RPI, z.B. der RPI1 aus *Ustilago maydis* angewendet werden.

Das Reaktionsvolumen betrug 700 µl. Zum Testpuffer (0,1 M Tris/HCl, pH8,0, 0,5 mM DTT) wurden entsprechende Volumina einer 50 mM Ribose-5-phosphat-Stammlösung zugegeben, und die Reaktion durch Zugabe von 1 µl der RPI1-Präparation (s. Beispiel 1) gestartet. Die Zunahme der Absorption bei einer Wellenlänge von 290 nm wurde spektrophotometrisch verfolgt. (Wood T., Assay for D-ribose-5-phosphate ketol isomerase and D-ribulose-5-phosphate 3-epimerase. *Methods Enzymol.* 41 (1975) 63-6; Wood, T., Spectrophotometric Assay for D-Ribose-5-phosphate Ketol-isomerase and for D-Ribulose-5-phosphate 3-Epimerase. *Analytical Biochemistry* 33 (1970) 297-306). Im Vergleich dazu wurde die Veränderung der Absorption bei Anwesenheit einer Kandidatenverbindung in variablen Konzentrationen beobachtet. Eine verringerte Absorption weist auf das Vorliegen eines Inhibitors der RPI hin.

### B) Diskontinuierlicher Enzymtest mit Carbazol und 75 % (v/v) Schwefelsäure

Der Test beruht auf dem Nachweis der Ketogruppe der Ribulose durch eine Farbreaktion mit einer Carbazol/Cystein/HCl-Lösung als Purpurfarbstoff (546 nm) (G.F. Domagk und K.M. Doering, *Methods in Enzymology* 41 (1975) 424ff; H. Horitsu, I. Sasaki, T. Kikuchi, H. Suzuki, M. Sumida und M. Tomoyeda. Purification, properties and structure of ribose 5-phosphate ketol Isomerase from *Candida utilis. Agr.Biol.Chem.* 40(2) (1976) 257-264). Der Reaktionsansatz in einem Ansatzvolumen von 50 µl besteht aus 5-10 mM Ribose-5-phosphat und 30 ng Enzym RPI, die für 15 min bei 37°C inkubiert werden. Dazu werden dann 5 µl einer Cystein-Lösung (0,03 M) sowie 30 µl 75 % (v/v) Schwefelsäure und 1 µl ethanolische 0,1 % (w/v) Carbazol-Lösung gegeben. Die enzymatische Reaktion wird anhand einer auftretenden Färbung verfolgt, die umso stärker ist, je mehr Substrat umgesetzt wird (Abb. 12). Bei Zugabe einer Kandidatenverbindung kann anhand der Abnahme der Absorption oder einfach anhand der geringeren Färbung eine inhibitorische Wirkung der Verbindung festgestellt werden.

### C) Gekoppelter Enzymtest mit Ribulose-5-phosphat-Kinase, PK und LDH

Der Nachweis einer enzymatischen Reaktion erfolgt hier fluorometrisch über die NADH-Abnahme (Che-Hun Jung, F.C. Hartman, Tse-Yuan, S. Lu, und Frank W.

Larimer. D-Ribose-5-phosphate Isomerase from Spinach: heterologous overexpression, purification, characterization, and site-directed mutagenesis of the recombinant enzyme. *Arch. Biochem. Biophys.* 373 (2000) 409). Dabei wird die interessierende Umsetzung von Ribose-5-phosphat zu Ribulose-5-phosphat mit weiteren enzymatischen Reaktionen gekoppelt, was letztlich zur Abnahme von NADH und damit zu einer Abnahme der relativen Fluoreszenz im Reaktionsverlauf führt (vgl. Abb. 13):

RPI
Ribose-5-phosphat → Ribulose-5-phosphat

Phosphoribulose-Kinase
Ribulose-5-phosphat + ATP → Ribulose-1,5-diphosphat + ADP

Pyruvat-Kinase
Phosphoenolpyruvat + ADP → ATP + Pyruvat

Lactat-Dehydrogenase
Pyruvat + NADH + H+ → Lactat + NAD+

Bei Zusatz einer Kandidatenverbindung zeigt sich deshalb keine oder eine verringerte Abnahme der relativen Fluoreszenz, wenn die getestete Verbindung eine Hemmung der enzymatischen Aktivität der RPI hervorruft.

### D) Gekoppelter Enzymtest mit Ribulose-5-phosphat-Epimerase, Transketolase, Glycerinaldehyd-3-phosphat-Dehydrogenase

Ribose-5-phosphat wird durch Ribose-5-phosphat-Isomerase in Ribulose-5-phosphat und dieses durch D-Ribulose-5-phosphat-Epimerase (EC 5.1.3.1) weiter zu Xylulose-5-Phosphat umgesetzt. In einer Transketolase-Reaktion (EC 2.2.1.1) dient dieses als Donor-Keton für den Acceptor-Aldehyd Ribose-5-phosphat, der in diesem Fall nicht extra zugesetzt wird, da er bereits als Substrat für das zu analysierende Enzym dient.

Man erhält somit pro mol Ribose-5-phosphat nur 0,5 mol Glycerinaldehyd-3-phosphat. Ribose-5-phosphat wird nur dann rasch umgesetzt, wenn große Mengen Ribose-5-phosphat-Isomerase zugegen sind. Glycerinaldehyd-3-phosphat wird dann durch GAP-DH weiter umgesetzt, wobei NAD⁺ zu NADH umgewandelt wird, so dass die Umsetzung von Ribose-5-phosphat zu Ribulose-5-phsophat letztlich zu einer Zunahme von NADH führt.

Die zu testenden Substanzen werden in 5 µl 5% (v/v) DMSO in einer 384-Mikrotiterplatte vorgelegt. Die Konzentration der Substanzen wird so bemessen, dass die Endkonzentration der Substanzen im durchgeführten Test 10 µM beträgt. Dazu werden 10 µl Enzymlösung (gekühlt bei 4°C) pipettiert. Die Enzymlösung ist wie folgt zusammengesetzt: 243 mM Imidazol-Puffer (pH 7,6), 27 mM MgCl₂, 0,02 % (w/v) Cocarboxylase (TPP), 0,1 % (w/v) BSA, 0,01 % (v/v) Tween 20, 90 mM NAD, 0,00038 u/µl Ribulose-5-phosphat-Epimerase aus Hefe (Sigma, Taufkirchen), 2,5 ng/µl isolierte RPI1 (s. Beispiel 2). Zu diesem Ansatz werden 20 µl der Substratlösung (gekühlt bei 4°C) gegeben, und damit die Reaktion gestartet. Die Substratlösung beeinhaltet 162 mM Imidazol-Puffer (pH 7,6), 18 mM MgCl₂, 0,03 %(w/v) Cocarboxylase (TPP), 0,075 % (w/v) BSA, 0,0075 % (v/v) Tween 20, 13,128 mM Ribose-5-phosphat, 0,5625 ng/µl Transketolase (in *E.coli* heterolog exprimierte Transketolase aus Mais), 0,00039 u/µl GAPDH (SIGMA, Taufkirchen), 10,8 mM NaH₂AsO₄. Es wird die Zunahme der Fluoreszenz bei λ= 360/35 nm (Extinktion) und λ=465/35 nm (Emission) bei Raumtemperatur für 3 h gemessen (wird in Vormessung ermittelt), wobei die Ergebnisse einer Messung bei Anwesenheit einer zu testenden Verbindung mit den Ergebnissen einer Messung bei Abwesenheit einer zu testenden Verbindung verglichen wurden. Als Kontrolle diente der oben beschriebene Ansatz in Abwesenheit der RPI1. Die im Test verwendeten Substanzen lagen dabei in folgenden Endkonzentrationen vor: c(Imidazol) = 162 mM, c(MgCl₂) = 18 mM, c(Cocarboxylase) = 0,03% (w/v), c(Ribose-5-phosphat) = 7,5 mM, c(NAD) = 25,71 mM, c(NaH₂AsO₄) = 6,17 mM, c(BSA) =0,071 % (w/v), c(Tween-20) = 0,007% (v/v), c (Transketolase) = 0,314 ng/µl, c(GAPDH) = 0,00022 U/µl, c (Ribulose-5-phosphat-3-Epimerase) = 0,000109 U/µl, m(RPI) = 0,714 ng/µl.

### Beispiel 4

### Nachweis der fungiziden Wirkung der identifizierten Inhibitoren der RPI

In die Kavitäten von Mikrotiterplatten wurde eine methanolische Lösung des anhand eines erfindungsgemäßen Verfahrens identifizierten Wirkstoffs (Tab. I) in der gewünschten Menge, versetzt mit einem Emulgator, pipettiert. Nachdem das Lösungsmittel abgedampft war, wurden je Kavität 200 µl Potatoe-Dextrose-Medium hinzugefügt. Das Medium wurde vorher mit geeigneten Konzentrationen von Sporen bzw. Mycelen des zu prüfenden Pilzes (siehe Tabelle II) versetzt.

Die resultierende Konzentration des Emulgators betrug 300 ppm.

Die Platten wurden anschließend auf einem Schüttler bei einer Temperatur von 22°C inkubiert, bis in der unbehandelten Kontrolle ein ausreichendes Wachstum feststellbar war. Die Auswertung erfolgte photometrisch bei einer Wellenlänge von 620 nm. Aus den Messdaten der verschiedenen Konzentrationen wird die Wirkstoffdosis, die zu einer 50 %igen Hemmung des Pilzwachstums gegenüber der unbehandelten Kontrolle führt (ED₅₀), berechnet (siehe Tab. II).

## Patentansprüche

1. Verfahren zum Identifizieren von Fungiziden, **dadurch gekennzeichnet, dass** man
(a) eine RPI oder eine Wirtszelle enthaltend dieses Polypeptid mit einer chemischen Verbindung oder mit einem Gemisch von chemischen Verbindungen unter Bedingungen in Kontakt bringt, die die Interaktion einer chemischen Verbindung mit dem Polypeptid erlauben,
(b) die Aktivität der RPI bei Abwesenheit einer chemischen Verbindung mit der Aktivität des erfindungsgemäßen Polypeptids bei Anwesenheit einer chemischen Verbindung oder eines Gemisches von chemischen Verbindungen vergleicht, und
(c) die chemische Verbindung bestimmt, die die Aktivität der RPI inhibiert.

2. Verfahren gemäß Anspruch 1, **dadurch gekennzeichnet, dass** man in einem folgenden Schritt (d) die fungizide Wirkung der bestimmten Verbindung prüft, indem man sie mit einem Pilz in Kontakt bringt.

3. Verwendung eines Polypeptids mit der biologischen Aktivität einer RPI, einer dafür kodierenden Nukleinsäure oder von Wirtszellen enthaltend dieses Polypeptid zum Identifizieren von fungiziden Verbindungen.

4. Verwendung eines Modulators eines Polypeptids mit der biologischen Aktivität einer RPI als Fungizid.

5. Verwendung eines Modulators eines Polypeptids mit der biologischen Aktivität einer RPI zur Bekämpfung von pflanzenpathogenen Pilzen.

6. Fungizide Modulatoren eines Polypeptids mit der biologischen Aktivität einer RPI, welche durch ein Verfahren gemäß Anspruch 1 oder 2 identifiziert werden.

7. Nukleinsäure, kodierend für ein Polypeptid aus pflanzenpathogenen Pilzen mit der biologischen Aktivität einer RPI.

8. Nukleinsäure gemäß Anspruch 7, **dadurch gekennzeichnet, dass** sie für eine RPI aus *U maydis* kodiert.

9. Nukleinsäuren gemäß Anspruch 7 oder 8, **dadurch gekennzeichnet, dass** es sich um einzelsträngige oder doppelsträngige DNA oder RNA oder um Fragmente genomischer DNA oder um cDNA handelt.

10. Nukleinsäuren gemäß einem der Ansprüche 7 bis 9, umfassend eine Sequenz ausgewählt aus
a) der Sequenz gemäß SEQ ID NO: 1,
b) Sequenzen, die für ein Polypeptid kodieren, welches die Aminosäuresequenz gemäß SEQ ID NO: 2 umfasst,
c) Sequenzen, die für ein Polypeptid kodieren, welches zumindest eine der Konsensussequenzen aus der folgenden Gruppe von Konsensussequenzen umfasst: -(I/V)GIGSGSTV-, -(I/V)D(I/V)X₂DGADE(I/V)DX₂LX₂IKGG-, -(P)TG(F/D)QSX₂LI-, - EK(V/L)X₄AX₂F(I/V)XVADX(R/S)K-, -WX₂G(I/V)PIEVXP-, -AKAGP(I/V)VTDNXNFX(W/L)D-, -IKXLXGVXEXGLF-, -AYFGNXDG-,
d) zumindest 15 Basenpaare langen Teilsequenzen der unter a) bis c) definierten Sequenzen,
e) Sequenzen, welche an die unter a) bis c) definierten Sequenzen bei einer Hybridisierungstemperatur von 42-65°C hybridisieren,
f) Sequenzen, welche eine 60 %, mindestens 65 %, mindestens 70 %, mindestens 75 %, mindestens 80 %, mindestens 85 %, mindestens 90 %, mindestens 95 % und insbesondere mindestens 98 % Identität mit den unter a) bis c) definierten Sequenzen aufweisen,
g) Sequenzen, welche zu den unter a) bis f) definierten Sequenzen komplementär sind, und
h) Sequenzen, welche aufgrund der Degeneriertheit des genetischen Codes für dieselbe Aminosäuresequenz codieren wie die unter a) bis c) definierten Sequenzen.

11. DNA-Konstrukt umfassend eine Nukleinsäure gemäß einem der Ansprüche 7 bis 10 und einen heterologen Promotor.

12. Vektor umfassend eine Nukleinsäure gemäß einem der Ansprüche 7 bis 10, oder ein DNA-Konstrukt gemäß Anspruch 11.

13. Vektor gemäß Anspruch 12, **dadurch gekennzeichnet, dass** die Nukleinsäure funktionell mit regulatorischen Sequenzen verknüpft ist, die die Expression der Nukleinsäure in pro- oder eukaryotischen Zellen gewährleisten.

14. Wirtszelle enthaltend eine Nukleinsäure gemäß einem der Ansprüche 7 bis 10, ein DNA-Konstrukt gemäß Anspruch 11 oder einen Vektor gemäß Anspruch 12 oder 13.

15. Polypeptid aus pflanzenpathpgenen Pilzen mit der biologischen Aktivität einer RPI, welches von einer Nukleinsäure gemäß einem der Ansprüche 7 bis 10 kodiert wird.

16. Verfahren zum Auffinden einer chemischen Verbindung gemäß Anspruch 1 oder 2, **dadurch gekennzeichnet, dass** man ein Polypeptid gemäß Anspruch 14 oder 15 verwendet.

17. Verfahren zum Auffinden einer Verbindung, welche die Expression von Polypeptiden gemäß Anspruch 14 oder 15 verändert, umfassend die folgenden Schritte:
(a) Inkontaktbringen einer Wirtszelle gemäß Anspruch 14 mit einer chemischen Verbindung oder einem Gemisch von chemischen Verbindungen,
(b) Bestimmen der Polypeptidkonzentration, und
(c) Bestimmen der Verbindung, welche die Expression des Polypeptids spezifisch beeinflusst.
